# EUROPEAN PATENT SPECIFICATION

(11) **EP 1 253 859 B1**
(45) Date of publication and mention of the grant of the patent: **15.04.2009**
(21) Application number: 01900873.9
(22) Date of filing: 03.01.2001
(51) Int. Cl.: A61B 17/11, A61B 17/32, A61B 17/34

(54) **APPARATUS FOR CREATING A CHANNEL BETWEEN ADJACENT BODY LUMENS**
VORRICHTUNG ZUR EINSTELLUNG EINES KANALS ZWISCHEN ANGRENZENDEN KÖRPERLUMEN
APPAREIL DESTINE A CREER UN CANAL ENTRE DES LUMIERES CORPORELLES ADJACENTES

(30) Priority: 04.01.2000 US 478109; 30.03.2000 US 539639
(43) Date of publication of application: 06.11.2002
(73) Proprietor: Medtronic Vascular, Inc., Santa Rosa, CA 95403 (US)
(72) Inventor: FLAHERTY, J., Christopher, Los Altos, CA 93022 (US); EVARD, Philip, C., Palo Alto, CA 94306 (US); ACOSTA, Pablo, G., Jr., Newark, CA 94560 (US); MACAULAY, Patrick, E., San Jose, CA 95118 (US); LAMSON, Theodore, C., Pleasanton, CA 94566 (US)
(74) Representative: Viering, Jentschura & Partner
(86) International application number: PCT/US2001/000266
(87) International publication number: WO 2001/049187

(56) References cited:
- EP-A- 0 473 790
- WO-A-92/14413
- WO-A-97/13463
- WO-A-97/13471
- WO-A-99/04704
- WO-A-99/51162
- US-A- 5 147 356
- PATENT ABSTRACTS OF JAPAN vol. 010, no. 183 (P-472), 26 June 1986 (1986-06-26) & JP 61 029733 A (YOICHI YAJIMA), 10 February 1986 (1986-02-10)

## Description

### Field of the Invention

The present invention relates to an apparatus for creating of channel between anatomical structures according to the preamble of claim 1. Accordingly, the present invention relates to apparatus for performing endoluminal procedures within a body lumen and between the body lumen and adjacent anatomical structures, such as nearby body lumens, and more particularly to apparatus and methods for accessing or creating channels between adjacent blood vessels, such as between coronary vessels or vessels of the peripheral vasculature, either during open surgical or endoluminal procedures.

### Background

Document WO-A-97/13463 which is considered as closest prior art discloses a catheter device with a tissue penetrating element for advancing out of the catheter in order to incorporate a guide wire. The cannula can be utilized as a conduit for introduction of drugs, for implantation of devices, for deployment of applications

Document US 5,147,356 discloses a surgical instrument having a prior of opposed jaw numbers, at least one of which jaw members is moveable with respect to the other jaw member. A cutting and/or cauterizing heat source such as a hot wire element and/or laser transmission fiber is carried an at least one of the jaw members.

### Background

It may be useful in a number of clinical applications to create a channel or passage through tissue between adjacent body lumens within a patient, such as between vessels in the cardiovascular system. Channels, created between anatomical structures may provide a therapeutic benefit to treat various diseases or other abnormal conditions of a patient. Channels may be created between blood vessels to provide oxygenated blood to an ischemic region. A channel between a space near the brain containing high pressure fluid and a nearby vein may be created to reduce the pressure and remove the fluid. Other vessels or structures that may experience restricted or blocked flow include blood vessels, vas deferens, fallopian tubes, intestines, lymphatic ducts, grafts, and chambers of the heart or brain.

For example, within the coronary system, a channel may be created between adjacent blood vessels to bypass an occlusion within a coronary artery as an alternative to traditional coronary bypass surgery. Using one or more endoluminal devices that may be advanced into the coronary vasculature from peripheral vessels, a channel may be created upstream of the occlusion between the coronary artery and a nearby coronary vein. The coronary vein may be blocked adjacent the channel such that blood from the coronary artery may pass through the channel and flow retrograde through the coronary vein, thereby providing revascularization of the heart without using the coronary artery downstream of the occlusion.

Alternatively, a channel may be created between the coronary artery and an adjacent vessel, such as a coronary vein, on either side of the occlusion, i.e., both upstream and downstream of the occlusion. The segment of the adjacent vessel may be isolated from its upstream and downstream portions, thereby allowing the vessel segment to be used to bypass the occlusion. Blood may then flow from the coronary artery upstream of the occlusion, through the first channel into the vessel segment and then through the second channel back into the coronary artery downstream of the occlusion. Such methods are shown, for example, in U.S. Patent No. 5,830,222 issued to Makower.

The channel may be created by ablating tissue between the adjacent vessels using laser energy, radio frequency (RF) energy, by mechanically cutting or boring a hole, by dilating a puncture opening, and the like. Clamping members, endoluminal grafts, or other tubular prostheses may be installed in or across the channel to help hold the channel open.

In these techniques, the operations are performed from within a blood vessel and generally from a remote location. For example, access to the vascular system may be gained from the femoral artery. Long catheters are introduced from the vascular access so that a distal end of the catheter is passed through the vasculature to the bypass site. The various cutting and vessel-to-vessel junctions are established at the bypass site using controls operated by the surgeon at the proximal end of the catheter (i.e. at the point of access to the vascular system). In some situations, however, such percutaneous procedures are difficult, if not impossible to perform, the patient may be involved in a simultaneous surgical procedure or the clinician may prefer a surgical approach and it is desirable to effect bypasses with an open surgical procedure.

In addition to coronary applications, it may be desirable to create a fistula or other channel between blood vessels in peripheral regions of a patient. For example, for a dialysis patient, a fistula may be created between the radial artery and the cephalic vein within the forearm. Once the fistula is created, the vein may enlarge in size over time, and, after maturation, may be routinely accessed for dialysis. Similar procedures may be used to revascularize other regions of the body, such as the lower legs. One of the problems associated with such fistulae is that they may not remain patent or provide sufficient flow for an indefinite period of time. Instead, the fistulae may quickly close up and heal after their creation, thereby requiring additional intervention or surgery to reopen or recreate the fistula.

Another problem with creating a simple tubular channel between two vessels is that the size of the channel may be limited by the size of the smaller of the two vessels, thereby limiting the use of this approach in relatively small vessels. In addition, since the channels traverse tissue that is not naturally vascular in nature, there is a necessary healing response that must occur for the channel to remodel and endothelialize. In a tubular channel to a small vessel, this healing process may result in severe narrowing or closure of the channel. Where the vessels are relatively far away from one another, a significant amount of non-vascular tissue may be present between the two vessels, and thus, the time for endothelium to grow in from the edges and cover the channel may be extensive. This longer period of pre-endothelialization may result in a higher degree of thrombosis or acute closure during the time required to close the endothelial gap.

Accordingly, it is believed that apparatus and methods for creating a channel between adjacent body lumens that facilitate placement or sizing of the channel, and/or that result in a channel that is more likely to remain patent would be useful.

### Summary of the Invention

To this end, the invention provides a wire-guided cutting assembly according to claim 1 and an apparatus according to claim 23. Further embodiments of the wire-guided cutting assembly of the present invention are described in the depended claims. The present invention is directed to apparatus for creating linear incisions or channels between anatomical structures, such as between adjacent blood vessels. In a preferred embodiment, an apparatus is provided that includes an elongate tubular member, such as a catheter, having proximal and distal ends defining a longitudinal axis therebetween. The catheter includes a distal portion adapted for insertion endoluminally into a body lumen, and a lumen for facilitating advancement of the tubular member over a guidewire. A cutting element is fixed at a predetermined location on a periphery of the distal portion, the cutting element configured for creating a linear incision in a wall of a body lumen substantially parallel to the longitudinal axis.

Preferably, the cutting element has a linear configuration on the distal portion oriented substantially parallel to the longitudinal axis. In one embodiment, the cutting element may be an electrode, such as a wire element that is disposed axially along the distal portion, the wire element including an exposed conductive region having a predetermined length. A source of electrical energy, such as an RF generator, may be coupled to the electrode for charging the electrode with sufficient electrical energy to cut tissue contacted by the electrode. Alternatively, the cutting element may be a mechanical cutter, such as a wire, a blade, or scissors.

A clip is provided adjacent the cutting element for engaging tissue between the clip and an outer surface of the elongate member for maintaining the cutting element in contact with the tissue. A sheath may be provided for selectively covering and uncovering the clip, the sheath maintaining the clip in close contact with the outer surface of the elongate member when the sheath covers the flexible clip. Alternatively, a filament may be connected to a loose end of the clip for releasably holding the loose end of the clip in close contact with the outer surface of the catheter. In a further alternative, the clip may be at least partially retractable into the elongate member to facilitate insertion of the catheter to a target site. An orientation element may be provided on the outer surface of the catheter and/or on the clip for rotationally orienting the cutting element and/or the clip.

In one embodiment, the distal portion of the catheter may include a pair of lateral portions, the lateral portions being movable between a closed position wherein the lateral portions are in contact with one another, and an open position wherein the lateral portions are disposed away from one another. The cutting element is located between the lateral portions, whereby the cutting element is exposed when the lateral portions assume their open position. Each of the lateral portions may have a lumen extending therethrough for facilitating advancement over a pair of guidewires.

The apparatus may be used for creating a channel through tissue, the channel extending from a first body lumen within a patient towards an anatomical structure, such as a second body lumen, beyond a wall of the first body lumen. An elongate member, such as the catheter or guidewire described above, may be advanced endoluminally into the first body lumen. A distal portion of the elongate member may be directed through a wall of the first body lumen towards the anatomical structure until a cutting element on the distal portion is disposed in contact with tissue therebetween. The elongate member may then be directed substantially axially along the first body lumen, thereby creating a linear incision in the tissue using the cutting element.

Before directing the elongate member substantially axially, the cutting element may be rotationally oriented along a desired cutting plane, preferably within which the first body lumen and the anatomical structure lie. The elongate member may be positioned such that a clip covering the cutting element engages tissue between the clip and an outer surface of the elongate member to enhance contact between the cutting element and the tissue.

In accordance with another aspect of the present invention, the apparatus may be used for sizing an interstitial channel through tissue within a patient is provided that includes creating a channel between two anatomical structures within the patient, such as between two blood vessels, monitoring a physical parameter associated with the patient after creating the channel to determine whether a desired effect has been achieved, and modifying the size of the channel in response to the physical parameter. If desired, the steps of monitoring the physical parameter and modifying the size of the channel may repeated until the desired effect has been achieved. In a preferred form, the physical parameter is a flow rate through the channel, and the desired effect is achieving a minimum flow rate through the channel. Alternatively, other physical parameters may be monitored, such as pressure, heart or respiratory function, and the like.

The apparatus of the present invention allow channels to be created between blood vessels and other anatomical structures that may have a larger cross-section than tubular channels. The length of the linear incision used to create the channel may be selected to be any desired length, thereby providing an effective diameter that may be substantially larger than tubular channels, which are limited in size to the smaller vessel being connected. This may substantially improve the patency of the channel and allow improved fluid flow therethrough.

In addition, channels created using the present invention may not have a direction bias, because the channels may be created substantially perpendicular to the course of the blood vessels. Such a configuration may be beneficial, particularly when the direction of the flow in the target vessel is different than that of the source vessel. Further, the perpendicular shape may facilitate the implantation of connectors or prostheses that may further improve the patency of the channel.

In accordance with another aspect of the present invention, apparatus may be provided for use in an open surgical field, e.g., for forming apertures in adjacent portions of anatomical structures, such as blood vessels, during a surgical procedure. The apparatus may be useful for creating alternative vascular channels to provide revascularization routes in the heart between the coronary arteries and between the cardiac veins, or in the periphery between adjacent veins, conduits, and/or arteries. They may also be used to bypass coronary arteries and to provide for cardiac venous arterialization. It will be appreciated by persons of ordinary skill in the art that the apparatus as described herein are equally applicable to the surgical manipulation of any anatomical structure for providing the passage of fluid flow therebetween. As used herein, the term "anatomical structures" or "body lumens" includes blood vessels, tubes, ducts, hollow organs, brain or heart chambers, grafts and synthetic structures.

An over-the-wire kit is described for forming apertures in adjacent portions of a first blood vessel and a second blood vessel during a surgical procedure. The kit includes a first guidewire for introduction into a lumen of the first vessel to an initial cutpoint, and then through adjacent portions of sidewalls of the first vessel and the second vessel into a lumen of the second vessel. As used herein, the phrase "initial cutpoint" refers to a position on the sidewall of the first vessel at which it is desired to establish a passage therethrough leading to the second vessel. The first guidewire is preferably introduced in an open surgical field, such as pursuant to a conventional open chest or minimally invasive procedure. The point at which the wire is introduced into the lumen of the first vessel may be near (or opposite) the initial cutpoint. Alternatively, the point of introduction may be a distance along the vessel from the initial cutpoint.

The kit also includes a wire-guided aperture-creating apparatus, which is configured to "follow" the wire, typically passing initially through a sidewall of a first vessel, through the lumen of that vessel to the initial cutpoint in the sidewall of the first vessel and then through the sidewalls of the first and second vessels, establishing apertures in those sidewalls. In one form, the aperture-creating apparatus includes a first elongated element extending along a first axis between a proximal end and a distal end. The aperture-creating apparatus also includes a second elongated element extending along a second axis between a proximal end and a distal end. The first elongated element is coupled to the second elongated element, whereby the first axis substantially intersects the second axis at a common intersection point spaced apart from the distal ends of the elongated elements. The distal end of the first elongated element is movable with respect to the distal end of the second elongated element about an axis orthogonal to the first axis and the second axis. A tissue cutting assembly is disposed between the first and second elongated elements. At least one of the first and second elongated elements includes a passage-defining element permitting passage therethrough of the first guidewire. In an alternate form, the kit includes a second guidewire, and the second elongated element also includes a passage-defining element, permitting passage of the second guidewire therethrough.

In one form, the cutting assembly is scissors-like, with the first and second elongated elements being rigid, and being pivotally joined at a common intermediate point, to form pivoting, opposed scissors-like blades on one side of the intermediate point and to form handles on the other side. At least one of the blades includes a channel for the first guidewire to pass through. The edges of the opposed elongated elements provide the tissue cutting assembly, as in conventional scissors.

The wire-guided aperture-creating apparatus may be formed from a flexible catheter with a bifurcated distal end, forming two opposed jaw-like elongated elements that are moveable in opposite directions about an axis transverse to the catheter at the point of bifurcation. The elongated elements may be flexible or rigid at least near their distal ends. In this form, the one (or both, in two-wire configurations) elongated element has a passage extending therethrough for passing over a guidewire. A tissue-cutting assembly is disposed between the elongated elements.

The tissue cutting assembly may include a blade positioned at the point of intersection or bifurcation, pointing away therefrom. Alternatively, one of the elongated elements may house a longitudinally extending blade and the other element may house an anvil, which, when the elongated elements are moved toward each other, may effect cutting of tissue therebetween. Alternatively, a laser cutter, or radio frequency (RF) cutter or hole punch (with anvil) may be positioned between the opposing elongated elements.

During use of the one (or two) wire kit described above, initially, in an open surgical field, a lead end of a first guidewire is passed through a veinotomy in a vein (the first blood vessel) and into its lumen. The lead end of the first guidewire is then advanced through the lumen until reaching a desired point (referred to as the initial cutpoint) of a junction between the first blood vessel and an adjacent artery (the second blood vessel). The lead end is then passed through the adjacent sidewalls of the first and second vessels and into the lumen of the second vessel. In a two-wire procedure, a second guidewire is passed through the veinotomy and into the lumen of the first blood vessel and is advanced so that its lead end is disposed within that lumen but extends beyond the initial cutpoint.

Following insertion of the guidewire(s), the path defining element of the first elongated element of the guided wire aperture-creating apparatus is passed over the first guidewire, and the distal ends of the elongated elements are fed through the veinotomy into the lumen of the first blood vessel. The wire-guided aperture-creating apparatus is advanced in the lumen, with the first elongated element tracking the first guidewire. Upon reaching the initial cutpoint, the first elongated element is led (by the first guidewire) from the lumen of the first blood vessel to the lumen of the second blood vessel, with the second elongated elements remaining in the lumen of the first blood vessel. In two wire procedures, the second elongated element is fed over the second guidewire at the same time that the first elongated element is fed over the first guidewire. Then, as the wire-guided aperture-creating apparatus is advanced just beyond the initial cutpoint, the first elongated element follows the first guidewire to the lumen of the second blood vessel, and the second elongated element affirmatively follows the second guidewire to continue in the lumen of the first blood vessel.

With the wire-guided aperture-creating apparatus so positioned just beyond the initial cutpoint, the tissue cutting assembly is then operated to cut tissue between the first and second elongated elements (i.e., simultaneously, the walls of the first and second blood vessels). In the scissors-like forms of the invention, handles can be operated to drive the blades to cut the tissue. In other forms, the various aperture-creating assemblies are operated to cut the tissue between the first and second elongated elements. For example, where there is an outwardly facing blade at the intersection or bifurcation point, the wire-guided aperture-creating apparatus need only be advanced so that the blade effects the cutting. For example, a laser or RF source near the distal end of one of the elongated elements might be actuated to ablate tissue between the elongated elements. Alternatively, the elongated elements may be driven toward each other so that a blade or punch on one elongated element cuts tissue between it and an anvil on the other elongated element. Following cutting a desired length, the wire-guided aperture-creating apparatus is withdrawn from the blood vessels and the adjacent vessels may be joined further, if necessary. A scaffolding device or other treatment may be provided to the aperture site for the purpose of maintaining fluid flow.

Other objects and features of the present invention will become apparent from consideration of the following description taken in conjunction with the accompanying drawings.

### Brief Description of the Drawings

For a fuller understanding of the nature and the objects of the invention, reference should be made to the following detailed description and the accompanying drawings in which like reference numerals refer to like elements and in which:
FIGS. 1A and 1B are side views of a first preferred embodiment of an apparatus for creating a linear incision between two adjacent body lumens.
FIG. 2 is a side view of a second preferred embodiment of an apparatus for creating a linear incision between two adjacent body lumens.
FIG. 3 is a side view of an apparatus for creating a linear incision between two adjacent body lumens.
FIGS. 4A and 4B are side views of a distal portion of a third preferred embodiment of an apparatus for creating a linear incision between two adjacent body lumens, showing the apparatus in a reduced profile and an enlarged profile, respectively.
FIGS. 4C and 4D are end views of the apparatus of FIGS. 4A and 4B, respectively.
FIG. 5 is a cross-sectional view of an arm, showing the apparatus of FIGS. 1A and 1B being used to create a channel between adjacent blood vessels in the arm.
FIG. 6A is a cross-sectional view taken along line A-A in FIG. 5, showing adjacent blood vessels and the rotational orientation of the apparatus with respect to a desired cutting plane within which the adjacent blood vessels lie.
FIG. 6B is a cross-sectional view of the blood vessels of FIG. 6A, showing the resulting linear incision created using the apparatus.
FIG. 6C is a cross-sectional view of the blood vessels of FIG. 6B, showing a tubular prosthesis implanted between the vessels to hold the channel open.
FIG. 7 is a cross-sectional view of an arm, showing the apparatus of FIG. 2 being used to create a channel between adjacent blood vessels in the arm.
FIGS. 8A and 8B are cross-sectional views of a leg, showing the apparatus of FIGS. 4A and 4B being used to create a channel between adjacent blood vessels in the leg.
FIGS. 9A-9D are cross-sectional views of adjacent blood vessels having an interstitial channel created between them.
FIG. 10 is a cross-sectional view of a distal portion of a fourth preferred embodiment of an apparatus for creating a channel between adjacent blood vessels.
FIG. 11 is a cross-sectional view of an arm, showing the apparatus of FIG. 10 being used to create a channel between adjacent blood vessels in the arm.
FIGS. 12A and 12B are side views of an apparatus having a retractable clip, showing the clip in retracted and extended positions, respectively.
FIG. 13 is a side view of an apparatus for creating a channel between adjacent blood vessels.
FIG. 14 is a cross-sectional view of adjacent blood vessels, showing the apparatus of FIG. 13 being used to create a channel therebetween.
FIG. 15A is a schematic representation of an over-the-wire kit configured for a "two wire" procedure, in accordance with the present invention.
FIG. 15B is a schematic representation of an alternate form of the over-the-wire kit configured for a "one wire" procedure, in accordance with the present invention.
FIG. 16A-16E illustrates a method for creating an aperture, in accordance with the present invention.
FIG. 17 is a schematic representation of an alternative wire-guided aperture-creating apparatus for inclusion in an over-the-wire kit, in accordance with the present invention.
FIGS. 18A-18E show variants of a wire-guided aperture-creating apparatus, in accordance with the present invention.
FIGS. 19A and 19B show deployments of a guidewire included in a kit, in accordance with the present invention.
FIG. 20 is a schematic representation of an over-the-wire aperture-creating apparatus with a single elongate member, including an aperture-creating element, an orientation marker, and a hemostasis apparatus.
FIG. 21 illustrates use of the single elongate member device between blood vessels.
FIG. 22 is a schematic representation of an over-the-wire aperture-creating apparatus with a single elongate member having an eccentric cutting edge.
FIG. 23A is a schematic representation of an over-the-wire aperture-creating apparatus with a single elongate member having a concentric channel creation element attached to an actuator.
FIG. 23B is a schematic representation of an over-the-wire aperture-creating apparatus with a single elongate member having an eccentric balloon attached to an actuator and an orientation marker.
FIG. 24 is a schematic representation of an over-the-wire aperture-creating apparatus with a single elongate member having a forward cutting distal tip.
FIG. 25 is a schematic representation of an over-the-wire aperture-creating apparatus with a single elongate member having a pull back cutting element and actuator with anvil.
FIG. 26 is a schematic representation of an over-the-wire aperture-creating apparatus with a single elongate member having a concentric balloon and actuator and, alternatively, a structural scaffold implant disposed thereon.
FIG. 27A is a schematic representation of an over-the-wire aperture-creating apparatus with a single elongate member having a concentric RF electrode and proximal connection.
FIG. 27B is a schematic representation of an over-the-wire aperture-creating apparatus with a single elongate member having an eccentric RF electrode and proximal connection including an orientation element.

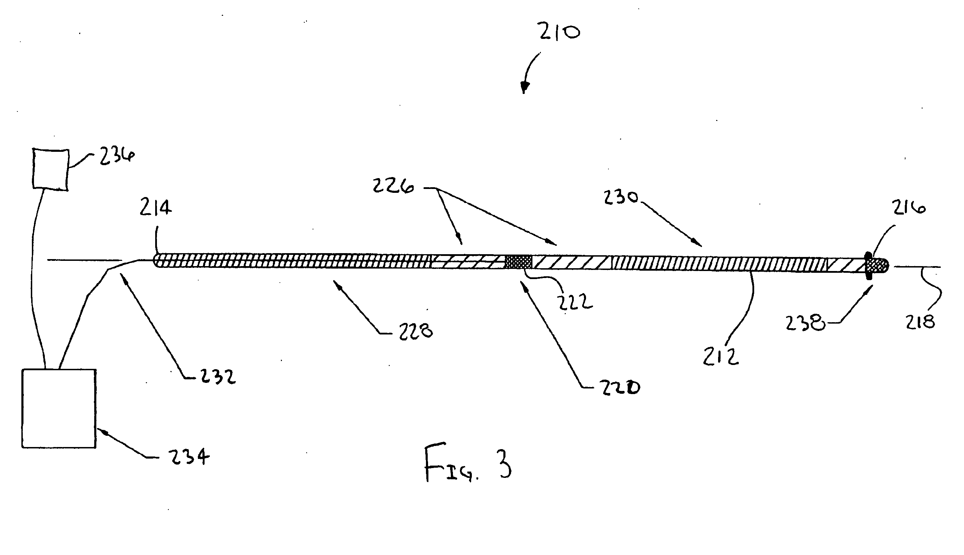

Figs. 3, 9A*-*9D*,* 13, 14, 20-27B do not show embodiments in accordance with the present invention.

### DETAILED DESCRIPTION OF THE PREFERRED EMBODIMENTS

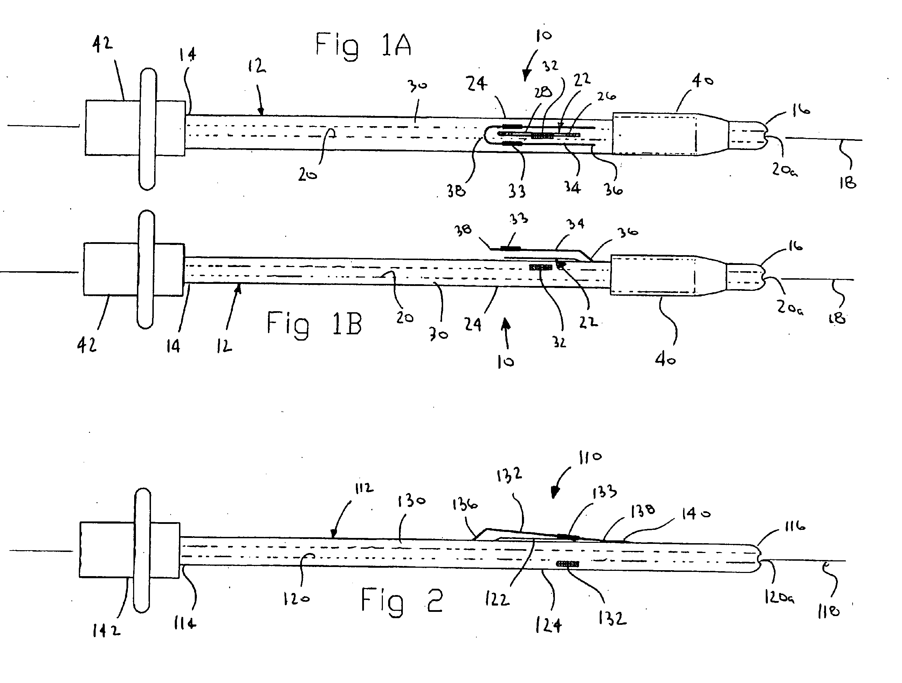

Turning to FIGS. 1A and 1B, a first preferred embodiment of an apparatus 10 is shown for creating a linear incision or channel in tissue between anatomical structures, such as adjacent body lumens, in accordance with the present invention. The apparatus 10 generally includes a catheter or other elongate member 12 having a proximal end 14, and a distal end 16, and having a cutting element 22 on a distal portion 24 thereof. The catheter 12 is preferably formed from one or more segments of a semi-rigid or substantially flexible tubular material, defining a longitudinal axis 18. The distal portion 24 of the catheter 12 preferably has a size and shape for facilitating insertion endoluminally into a body lumen, such as a vein or other blood vessel. The distal portion 24 may include flexible regions (not shown) with the cutting element 22 provided between the flexible regions. Thus, the distal portion 24 may be configurable in a generally serpentine or "S" shape to facilitate its placement between adjacent body lumens, as described further below.

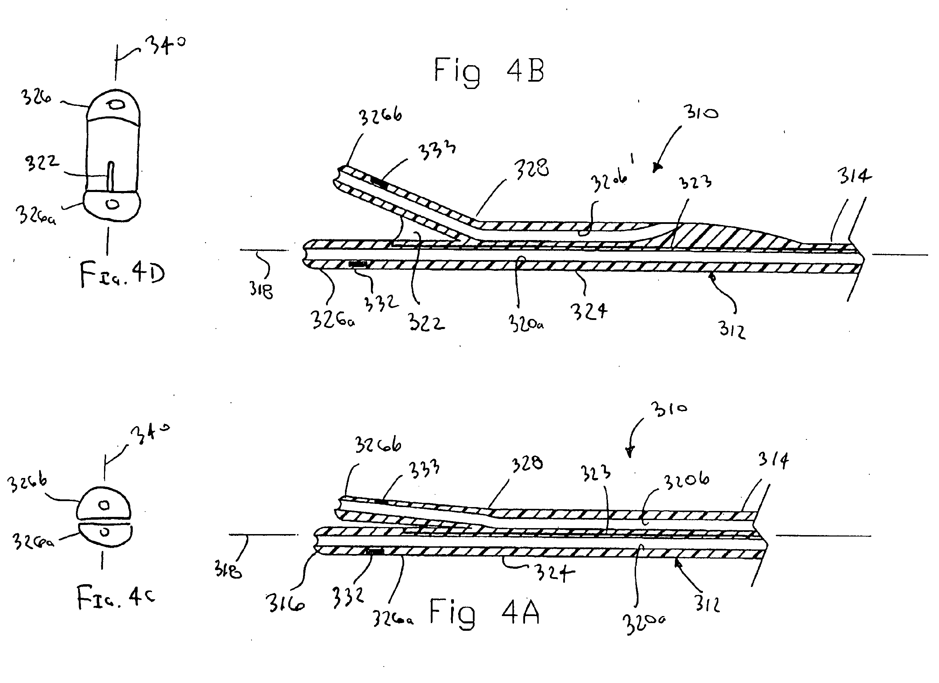

Preferably, the catheter 12 also includes a guidewire lumen 20 extending between the proximal and distal ends 14, 16 for facilitating advancement of the catheter 12 over a guidewire (not shown). Alternatively, the guidewire lumen 20 may have an outlet port 20a at the distal end 16 of the catheter 12, and an inlet port (not shown) at a predetermined peripheral location proximal to the outlet 20b, i.e., between the distal portion 24 and the proximal end 14, thereby providing a rapid exchange feature, as is known to those skilled in the art. An example of a rapid exchange guidewire lumen is shown in FIG. 4B.

Returning to FIGS. 1A and 1B, the cutting element 22 preferably has a linear configuration oriented substantially parallel to the longitudinal axis 18 of the catheter 12. In one preferred embodiment, the cutting element 22 is an electrode, such as a filament or wire 26 having an exposed conductive region 28. The wire 26 may be mounted axially along an outer surface 30 of the catheter 12 at a predetermined location about a periphery of the catheter 12. Alternatively, the wire 26 may be molded or otherwise inset into the surface 30 of the catheter 12, such that at least the conductive region 28 is exposed thereon. Other electrode structures may be provided on the distal portion 24, such as an annular band of material having an exposed conductive region, a conductive film having a predetermined length and width, and the like.

A conductor, such as an insulated wire, mandrel, or metallic braid (not shown), is coupled to the electrode 22 that extends proximally within the catheter 12 to the proximal end 14. The proximal end 14 of the catheter 12 may include a connector (not shown) coupled to the conductor, thereby facilitating connection to a source of energy, such as a radio frequency (RF) generator (not shown). The RF generator may include a second electrode, such as a pad (not shown), that may be disposed externally on the patient's skin, thereby allowing the generator to be operated in a unipolar mode, as is well known in the art. Alternatively, a second electrode (not shown) may be provided on the catheter 12, for example, adjacent the cutting element 22, or on other structures associated with the catheter 12, thereby allowing the generator to be operated in a bipolar mode.

In an alternative embodiment, the cutting element 22 may be a mechanical cutter (not shown). For example, a thin cross-section wire or blade (not shown), possibly serrated along a portion or all of its length, may be mounted to the outer surface 30 of the distal portion 24, having a predetermined length extending substantially parallel to the longitudinal axis 18. In a further alternative, a scissors device (not shown) may be provided on the distal portion 24 that includes one or more movable mandibles for cutting tissue engaged between the mandibles or between a mandible and a fixed blade on the distal portion.

A first orientation element 32 is preferably provided on the distal portion 24 in a predetermined peripheral relationship with the cutting element 22. For example, the first orientation element 32 may include one or more radiopaque markers on the outer surface 30 of the catheter 12, thereby facilitating determining the rotational orientation of the cutting element 22 using fluoroscopy. Such markers are disclosed in WO 97/13463. Alternatively, one or more ultrasound-reflective markers may be provided. In a further alternative, an imaging element, such as an intravascular ultrasound ("IVUS") device (not shown), may be provided on or in the distal portion 24 in addition to or instead of the radiopaque markers. The IVUS device may allow imaging along a plane substantially perpendicular to the longitudinal axis 18, for example, for providing a rotational orientation of the catheter 12 with respect to anatomical landmarks within tissue surrounding the apparatus 10 when it is disposed within a body lumen.

A tissue clip 34 is preferably provided on the distal portion for enhancing contact between the cutting element 22 and tissue being cut, i.e., by substantially engaging the tissue between the clip 34 and the outer surface 30 of the catheter 12. The clip 34 may be formed from a wire or other filament that may be looped or shaped to provide a desired rigidity and/or resilient flexibility. Alternatively, a tongue or substantially flat sheet of metallic or plastic material may be formed in a desired shape and mounted to the catheter 12. In a preferred embodiment, the clip 34 has a fixed end 36 attached to the distal portion 24 distal to the cutting element 22, and a loose end 38 disposed proximal to the cutting element 22. The fixed end 36 is preferably flexible and biased to direct the loose end 38 a predetermined distance from the outer surface 30 of the catheter 12. If it is desired to operate the apparatus 10 in a bipolar mode, a second electrode (not shown) may be provided on the loose end 38 or elsewhere on the clip 34.

Optionally, a second orientation element 33 may be provided on the clip 34, in addition to the first orientation element 32 on the outer surface 30 of the catheter 12, to further facilitate orientation of the apparatus 10. Preferably, the second orientation element 33 is placed on or near the loose end 38 of the clip 34. The second orientation element 33 may be monitored externally, for example, using fluoroscopy, to observe whether the clip 34 is properly deployed and/or to verify that tissue is properly engaged by the clip 34, as described further below.

A sheath 40 is provided that is slidable on the distal portion 24 of the catheter 12 for selectively covering and uncovering the clip 34 and/or the cutting element 22. The sheath 40 may be coupled to a cable and the like (not shown) that extends to a handle 42 on the proximal end 14 of the catheter 12. The handle 42 may be directed axially, i.e., distally and proximally, to advance and retract the sheath 40 to expose or cover the clip 34, respectively. In the proximal or covered position (not shown), the sheath 40 substantially covers the clip 34 and preferably maintains the clip 34 in close contact with the outer surface 30 of the catheter 12, thereby minimizing the profile of the apparatus 10. When the sheath 40 is directed to its distal position (shown in FIGS. 1A and 1B), the clip 34 is exposed and preferably resiliently extends out partially, for example, such that it extends substantially parallel to the longitudinal axis 18 of the catheter 12.

Alternatively, the clip 34 may be selectively secured and released from contact with the outer surface 30 of the catheter 12. For example, a pullwire or other filament may be directed through a hole (not shown) in the loose end 38 of the clip 34 that may then be directed into a lumen (also not shown) in the catheter 12 and to the proximal end 14 of the catheter 12. The ends of the pullwire may be drawn proximally or tightened from the proximal end 14 of the catheter 12 to pull the loose end 38 against the outer surface 30 of the catheter 12. When it is desired to expose the clip 34, the pullwire may be released and/or withdrawn from the hole by pulling one end of the pullwire proximally from the proximal end 14 of the catheter 12 until the other end of the pullwire exits the hole in the loose end 38, whereupon the clip 34 may resiliently extend outwardly, e.g., such that the loose end 38 is disposed a predetermined distance from the outer surface 30 of the catheter 12.

In a further alternative, the loose end 38 of the clip 34 may be releasably secured to the catheter 12 using a latching mechanism (not shown) that may be actuated from the proximal end 14 of the catheter 12. Alternatively, a sheath or guide catheter (not shown) may be provided that is slidable over the entire distal portion 24 of the catheter 12, for example, from the proximal end 14 for selectively covering and/or exposing the clip 32 and/or cutting element 22.

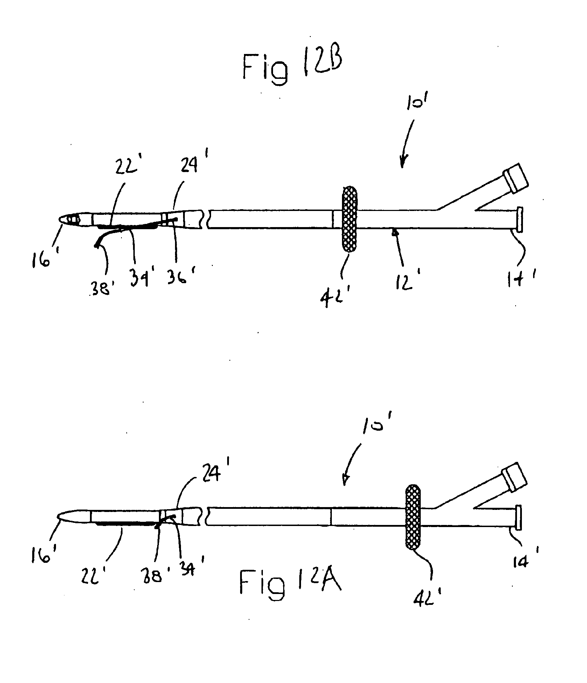

In still a further alternative, shown in FIGS. 12A and 12B, the clip 34' may be retractable into and/or extendable from slots or a cavity (not shown) in the catheter 12'. For example, an actuating handle 42' or other control mechanism may be provided on a proximal portion 14' of the catheter 12' that is coupled to the clip 34'. The clip 34' preferably includes a pair of slidable ends 36' that are coupled to the actuating handle 42', for example, by cables (not shown) within the catheter 12. The loose end 38' of the clip 34' is a U-shaped loop that extends from the fixed ends 36'. When the handle 42' is positioned proximally, the clip 34' is retracted, with the loose end 38' drawn in close against the outer surface 30' of the catheter 12' (FIG. 12A). The handle 42' may be directed distally, whereupon the clip 34' is extended out and away from the outer surface 30' of the catheter 12', for example, once the distal portion 24 is positioned as desired within the crossing site 86. The clip 34' may then be used to engage tissue in contact with the cutting element 22' or to align the apparatus 10' during creation of the channel. The clip 34' may then be retracted again to facilitate withdrawal of the apparatus from the patient's body.

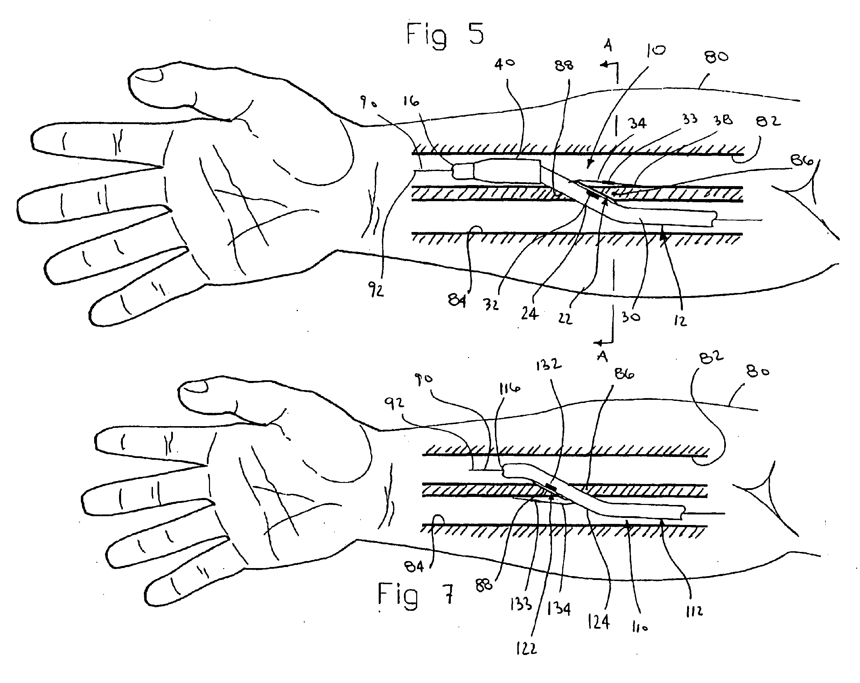

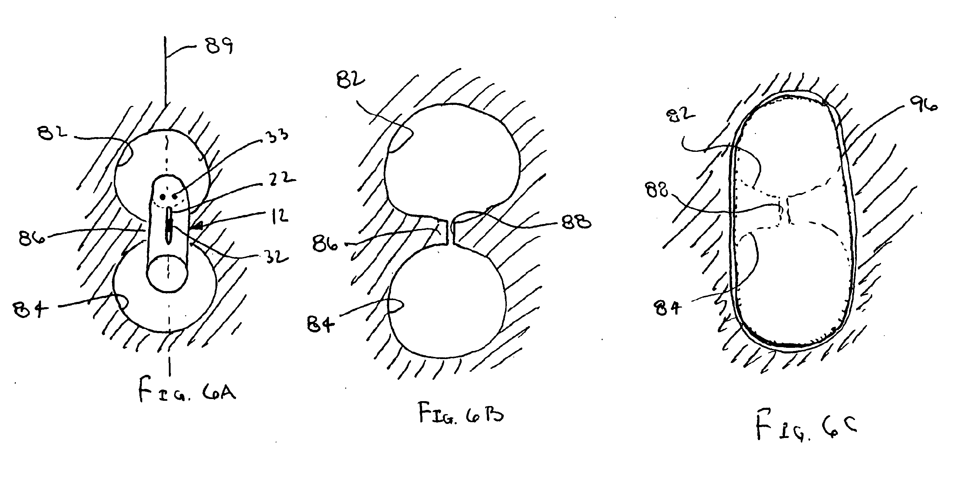

Turning to FIGS. 5, 6A, and 6B, the apparatus 10 may be used to create a linear incision, channel, or fistula 88 at a crossing site 86 between an artery 82 and a vein 84, such as the radial artery and the cephalic vein in a forearm 80 of a patient. The crossing site 86 may be any appropriate location for creating the fistula 88, which may be used to modify the vein, e.g., enlarge its cross-section. Once the fistula 88 matures, the resulting length of vein 84 may support repeated needle access to perform dialysis procedures , e.g., removal, filtering, and returning of blood to and from the patient's vasculature from a dialysis machine (not shown), as is well known in the art. The examples described herein refer to a vein being the starting vessel, i.e., the vessel into which the apparatus 10 is initially advanced, and an artery being the target vessel, i.e., the vessel that is the intended destination for the fistula or interstitial channel being created. Alternatively, an artery may be the starting vessel and a vein the target vessel, as will be appreciated by those skilled in the art. It will also appreciated that other body lumens or anatomical structures may also be the target location for the channel.

First, a guidewire or other rail 90 may be directed into the vein 84, and placed through the intervening tissue at the crossing site 86 into the artery 82. US-A-6 379 319 discloses devices and methods for placing guidewires across and/or between adjacent blood vessels. For example, the guidewire 90 may originate from a percutaneous entry site within a peripheral vein, such as an internal jugular vein or femoral vein, through the patient's vasculature into the vein 84, and finally through the intervening tissue 86 into the artery 82 such that a distal tip 92 of the guidewire 90 is located distally (with respect to the vasculature, "distally" is used herein to refer to locations further away from the heart, and "proximally" to locations closer to the heart) of the crossing site 86.

With the sheath in its covered position (not shown), the apparatus 10 may be advanced over the guidewire 90 from the entry site until the distal portion 24 enters the vein 84, and then advanced further such that the guidewire 90 guides the distal portion 24 through the crossing site 86 and into the artery 82. Preferably, the distal end 16 of the catheter 12 has a substantially rounded or pointed tip to facilitate its insertion through the intervening tissue at the crossing site 86 in a substantially atraumatic manner. With the distal portion 24 disposed within the artery 82, the sheath 40 is advanced to its distal position, exposing the clip 34 within the artery 82. Once the clip 34 is exposed, it preferably deflects away from the outer surface 30 of the catheter 12, providing a predetermined distance or gap between the loose end 38 of the clip 34 and the outer surface 30 of the catheter 12 for engaging tissue therebetween.

Before or after exposure of the clip 34, the apparatus 10 may be rotationally oriented, i.e., rotated about the longitudinal axis 18, to orient the cutting element 22 and the clip 34 away from the crossing location 86 and/or into a desired cutting plane. Preferably, as best seen in FIG. 6A, the cutting element 22 and the clip 34 are preferably aligned in a cutting plane 89 within which the artery 82 and the vein 84 lie, i.e. a plane substantially tangential to the longitudinal axis of the artery 82 and/or vein 84. To assist in orienting the apparatus 10, external imaging, such as fluoroscopy or ultrasound, may be used to monitor the marker(s) 32, 33 and thereby determine the orientation of the cutting element 22 and the clip 34. The, marker(s) 32, 33 may identify an axial position of the distal portion 24 within the vein 84, e.g., to align the distal portion 24 with respect to the crossing site 86. The marker(s) 32, 33 may also be used to identify that the cutting element 22 is properly in contact with tissue, for example, by observing the spreading of the markers 32, 33 away from each other as tissue is engaged under the clip 34.

Alternatively, if an IVUS device (not shown) is provided on the apparatus 10, it may be activated to image substantially perpendicular to the longitudinal axis 18, and thereby monitor the orientation of the cutting element 22 and/or the clip 34 with respect to the desired cutting plane 89. The cutting element 22 and/or the clip 34 may be at least partially ultrasonically reflective or other ultrasonic markers, such as a cage structure, may be provided on the distal portion 24 that is aligned axially with the cutting element 22 along the periphery of the catheter 12. Thus, the IVUS device preferably allows observation of these markers or structures with respect to the surrounding anatomical structures, such as the artery 82 and vein 84 themselves, to properly align the cutting element 22 and/or clip 34 within the plane 89.

Returning to FIG. 5, once properly rotationally aligned, the apparatus 10 may then be directed proximally, i.e., partially withdrawn back into vein 84. During this action, the clip 34 preferably slidably engages the wall of the artery 82, thereby engaging the intervening tissue at the crossing site 86 between the clip 34 and the cutting element 22. Because of its resilient flexibility, the clip 34 preferably freely slides along the wall of the artery 82, yet applies sufficient force to ensure contact of the intervening tissue 86 with the cutting element 22. The clip 34 may also provide a tactile indication that the cutting element 22 has been properly positioned across the intervening tissue at the crossing site 86, e.g., when the fixed end 36 of the clip 34 abuts the intervening tissue 86 adjacent to the artery 82.

Once the distal portion 24 is properly positioned across the crossing site 86, the cutting element 22 preferably extends completely across the intervening tissue 86 between the artery 82 and the vein 84. More preferably, the cutting element 22 is aligned substantially proximally, i.e., upstream with respect to the artery 82. If the cutting element 22 is an electrode and the like, it may be activated, for example, by driving it with sufficient RF energy to ablate and destroy tissue contacted by it. The apparatus 10 may then be directing substantially proximally, i.e., substantially parallel to the vein 84, thereby destroying and cutting through the intervening tissue 86 along the plane within which the artery 82 and vein 84 lie. Alternatively, if the cutting element 22 is a mechanical cutter, such as a wire or blade, the apparatus 10 may simply be retracted substantially proximally such that the cutting element 22 slices through any tissue engaged between the clip 34 and the cutting element 22, or the scissors (not shown) may be activated to periodically cut through the intervening tissue 86. In addition to ensuring contact between the cutting element 22 and the tissue being cut, the clip 34 may also act as a guide to ensure that the cutting element 22 remains substantially within the desired cutting plane.

Thus, the cutting element 22 preferably creates a linear incision or channel 88 in the intervening tissue proximal to the crossing site 86 that preferably extends completely between the artery 82 and the vein 84, as shown in FIG. 6B. The channel 88 has a length that may be precisely controlled, for example, by monitoring and measuring the distance that the apparatus 10 is retracted, for example, from the percutaneous entry site. Consequently, the size of the channel may be controlled more directly, and may allow substantially longer and therefore greater cross-section channels to be created between adjacent blood vessels.

In alternative methods, the apparatus 10 may be used to create a linear incision or channel between other anatomical structures within a patient. For example, the apparatus 10 may be used to create a channel within the coronary system, such as between a coronary artery having an occlusion therein and an adjacent coronary vein (not shown). A channel may be created upstream of the occlusion between the coronary artery and a nearby coronary vein. The coronary vein may be blocked adjacent the channel such that blood from the coronary artery may pass through the channel and flow retrograde through the coronary vein, thereby providing revascularization of the heart without using the coronary artery downstream of the occlusion. Alternatively, a channel may be created between the coronary artery and an adjacent vessel, such as a coronary vein, on each side of the occlusion, i.e., both upstream and downstream of the occlusion. The segment of the adjacent vessel may be isolated from its upstream and downstream portions, thereby allowing the vessel segment to be used to bypass the occlusion. Blood may then flow from the coronary artery upstream of the occlusion, through the first channel into the vein segment, and then through the second channel back into the coronary artery downstream of the occlusion.

If desired, once a channel 88 of an initial size is created between two anatomical structures, for example, between the two blood vessels 82, 84 of FIG. 5, a physical parameter associated with the patient may be monitored to determined whether a desired effect has been achieved. For example, a flow rate through the channel 88, through the artery 82, and/or through the vein 84 may monitored to ensure that a channel 88 of sufficient size is created in order to obtain a predetermined minimum flow rate therethrough. To measure this flow rate, a flow monitoring element (not shown) may be provided on the distal portion 24 of the catheter 12. Alternatively, a pressure sensor (not shown) may be provided on the apparatus 10 for measuring pressure within the channel 88 or vessels 82, 84, for example, to ensure that a predetermined maximum pressure is not exceeded (e.g., if it is desired to relieve pressure within an anatomical structure) or to detect a desired blood pressure level (e.g., to maintain a desired blood pressure level within the channel). Exemplary sensors that may be incorporated into an apparatus in accordance with the present invention are disclosed in U.S. Patent Nos. 4,947,852 issued to Nassi et al., and 5,715,827 issued to Corl et al. In further alternatives, other sensors (not shown) may be provided on the apparatus 10, separately within the blood vessels, or externally to the patient for monitoring other physical conditions in conjunction with the creation of the channel 88, such as EEG, EKG, respiratory rate, oxygen content or saturation, cardiovascular performance, visual clinical observations, such as skin color, or other physiologic, mechanical, or fluid dynamic properties that may be modified by the new condition.

If, based upon the measured parameter, it is determined that the channel 88 is too small (e.g., if the flow rate is too low), the apparatus 10 may be retracted further proximally to enlarge the length of the channel 88. The physical parameter may again be monitored, and these steps repeated until a desired effect (e.g., flow rate) is achieved. If it is determined that the channel 88 is too large, a covered stent or other endoluminal prosthesis (not shown) may be implanted to partially block the channel 88 and thereby reduce its effective size.

Once the channel 88 is created, the apparatus 10 may be directed distally, i.e., to advance the distal portion 24 back into the artery 82 and thereby disengage any intervening tissue 86 from under the clip 34. The sheath 40 may then be retracted to its covered position, thereby substantially covering the clip 34 and/or directing the clip 34 against the outer surface 30 of the catheter 12 to prevent tissue from subsequently becoming engaged by the clip 34. The apparatus 10 may then be withdrawn from the artery 82 through the channel 88, from the vein 84, and preferably from the patient's body. If desired, as shown in FIG. 6C, a tubular prosthesis 96, connector or other structure may be implanted within or across the channel 88 to improve the patency of the channel 88 and/or to trap any loose tissue in the channel 88 from being exposed to blood flow. Such prostheses and methods for implanting them are disclosed in PCT Publication No. WO 97/1347. The guidewire 90 may be used during the implantation of the prosthesis and/or withdrawn from the patient before or after its implantation.

Turning to FIG. 2, a second preferred embodiment of an apparatus 110 for creating a linear incision is shown that includes a catheter 112 having a proximal end 114, a distal end 116, and defining a longitudinal axis 118 therebetween, similar to the embodiment described above. A cutting element 122 is provided on a distal portion 124 of the catheter 112, such as an electrode or a mechanical cutter, and a tissue clip 134 is provided on the distal portion 124 that is disposed across cutting element 122, similar to that described above. If desired, first and second orientation elements 132, 133 may be provided on the outer surface 130 of the catheter 112 and the clip 134 to facilitate orienting the apparatus 110, similar to the embodiment described above.

Unlike the previous embodiment, the fixed end 136 of the clip 134 is located proximal to the cutting element 122, and the loose end 138 extends distally beyond the cutting element 122. In addition, a filament 140 or other physical restraint (not shown) is provided that may used to secure the loose end 138 of the clip 134 against the catheter 12, such as the pullwire described above. Thus, while the previous embodiment was a "pull back" slicer, the apparatus 110 is a "push forward" slicer, as described below.

Turning to FIG. 6, the apparatus 110 may be used to create a channel 88 between adjacent body lumens, such as the radial artery 82 and the cephalic vein 84 within the forearm 80. With the clip 134 secured by the filament 140 (see FIG. 2) against the catheter 112, the apparatus 110 is advanced over a guidewire 90 previously placed between the artery 82 and the vein 84 at a crossing site 86 until the distal portion 124 enters the vein 84. The clip 134 may be deployed, e.g., released from the filament 130, for example, by pulling a loose end (not shown) of the filament 130 from the proximal end 114 of the catheter 112. The clip 134 and/or cutting element 122 may be rotationally oriented within the vein 84 to align them with a desired cutting plane within which the artery 82 and vein 84 lie, e.g., to direct them away from the artery 82, by imaging the orientation elements 132, 133, as described above.

The apparatus 110 may be directed distally, such that the distal portion 124 advances through the intervening tissue at the crossing site 86. The clip 134 preferably slides along the wall of the vein 84 until the fixed end 138 abuts the intervening tissue 86, engaging the intervening tissue 86 under the clip 134. The cutting element 122 may be activated, if necessary, and the apparatus 110 directed distally, i.e., in a direction parallel to the vein in an upstream direction, whereupon the cutting element 122 slices through the intervening tissue contacted by the cutting element 122 distal to the crossing site 86. The apparatus 110 may be advanced a predetermined distance, thereby creating a linear incision or channel 88 having a length substantially equivalent to the predetermined distance. The apparatus 110 may then be withdrawn, a tubular prosthesis, connector, and the like (not shown) may be implanted, and/or the guidewire 90 withdrawn, similar to the embodiment described above. Alternatively, a physical parameter associated with the patient may be monitored and the size of the channel 88 modified accordingly, similar to the embodiment described above.

Turning to FIG. 3, an apparatus 210 for creating a linear incision or channel in tissue between anatomical structures is shown. The apparatus 210 includes a guidewire or other elongate member 212 having a proximal end 214, a distal end 216, and defining a longitudinal axis 218. A cutting element 222 is provided at an intermediate portion 224 between adjacent flexible or "floppy" portions 226, which are, in turn, adjacent to semi-rigid or "less floppy" proximal and distal portions 228, 230.

Preferably, the cutting element 220 comprises an electrode 222 having a predetermined length. The electrode 222 is coupled to a conductor 232 on the proximal end 214 of the guidewire 212, which is, in turn, coupled to an RF generator 234 or other source of electrical energy. An external electrode 236 is also coupled to the RF generator 234 for placement in contact with the patient's skin for providing a return path for the electrical energy delivered through the electrode 222. Alternatively, a mechanical cutter (not shown) may be substituted for the electrode 222, although the guidewire 212 may then require a sheath (not shown) for selectively exposing the mechanical cutter when it is desired to cut tissue.

The distal end 216 of the guidewire 212 preferably includes a snaring element 238 for securing the distal end to an endoluminal snaring device. Such snaring elements are disclosed in US-A-6 379 319

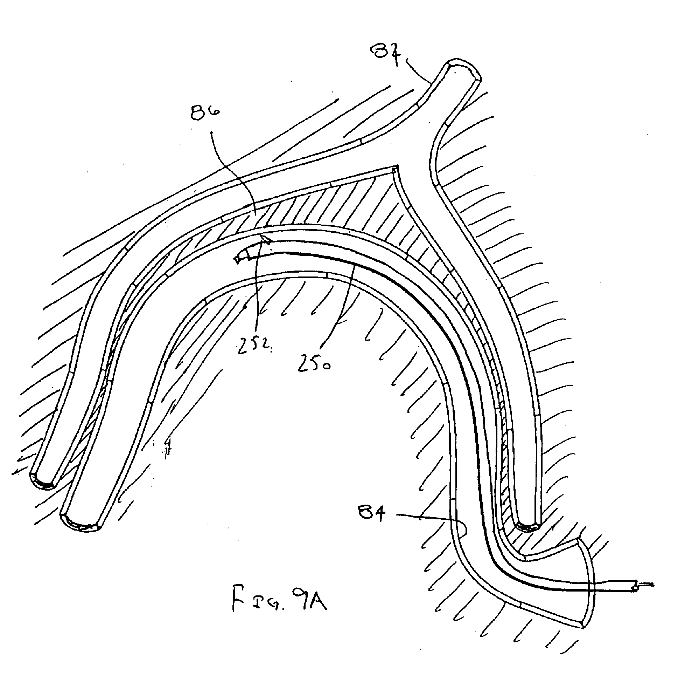

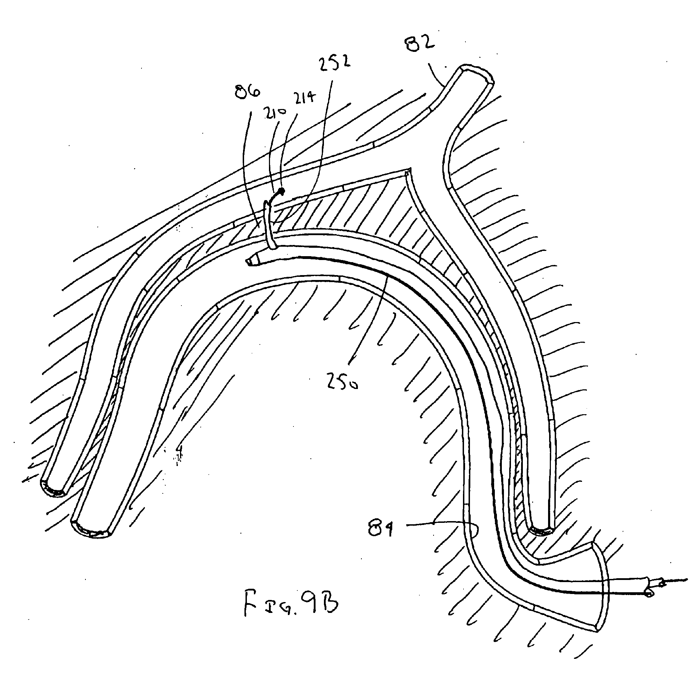

As shown in FIGS. 9A-9D, the apparatus 210 may be used to create a channel 88 between two blood vessels 82, 84, for example, within the coronary system. The apparatus 210 may be advanced from a first percutaneous entry site (not shown) along a first blood vessel 82 and directed through tissue at a crossing site 86 into a second blood vessel 84. First, a delivery device 250 having a peripherally deployable needle 252 may be advanced from the percutaneous site into the first blood vessel 82 adjacent to the crossing site 86 (FIG. 9A). The needle 252 may be oriented and deployed from the delivery device 252 through the tissue at the crossing site 86 and into the second body lumen. The apparatus 210 may the be advanced distally through the needle 252 from the proximal end (not shown) of the delivery device 250 until its distal end 216 exits the needle 252 and is disposed within the second blood vessel (FIG. 9B). The delivery device 250 may then be withdrawn, leaving the apparatus 210 extending between the first and second blood vessels 82, 84 at the crossing site 86.

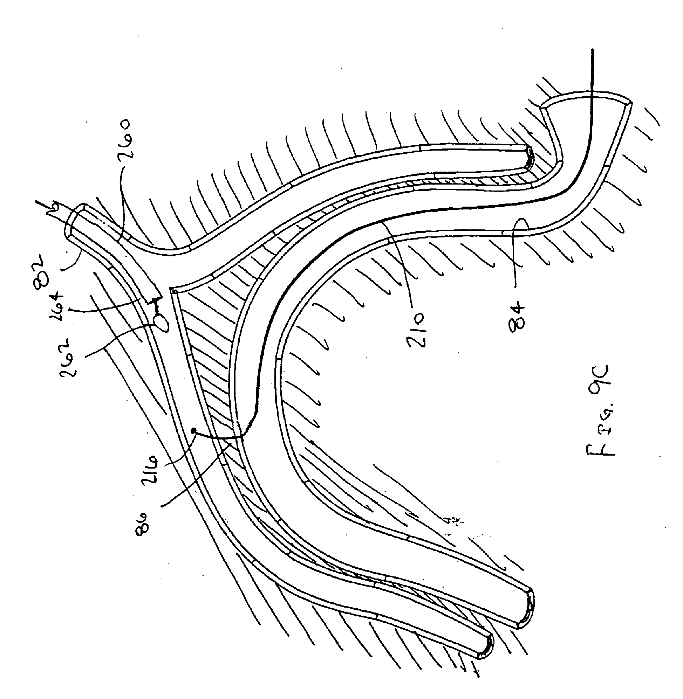

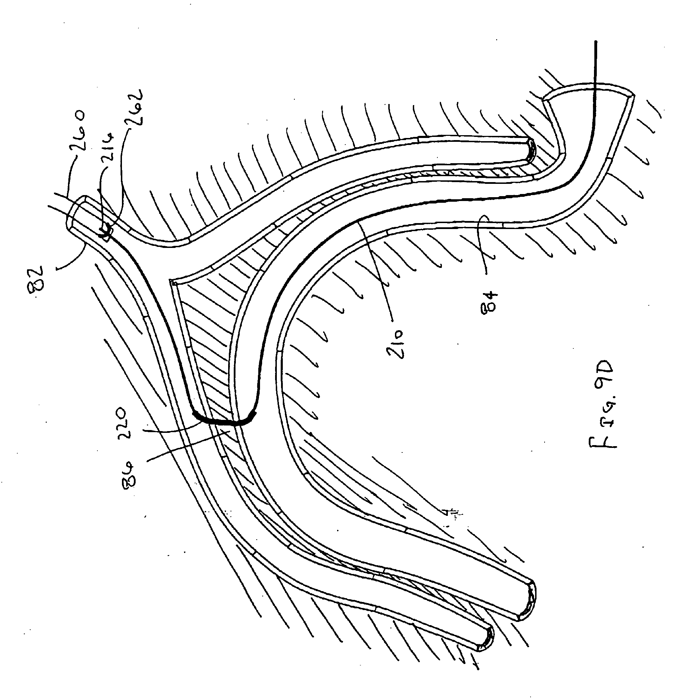

A snaring member 260 may be advanced from a second percutaneous entry site (not shown) along the second blood vessel 82 towards the crossing site 86 (FIG. 9C). The snaring member 260 preferably has a snare 262 on its distal end 264 that may be used to capture the distal end 216 of the apparatus 210. Preferably, the distal end 216 of the apparatus 210 has a snaring element, such as a bulbous tip, that may be securely received in the snare 262. Alternatively, other mating elements may be provided on the distal end 216 of the apparatus 210 and/or on the distal end 264 of the snaring member 260, such as those disclosed in US-A-6379319. The snaring member 260 may then be withdrawn from the second blood vessel 82 (FIG. 9D), thereby pulling the distal end 216 of the apparatus 210 from the second blood vessel 82, and preferably out of the patient's body.

The apparatus 210 may then be manipulated to position the cutting element 220 within the tissue at the crossing site 86 and preferably extending completely across between the first and second blood vessels 82, 84. This manipulation may be assisted by observing one or more orientation elements (not shown) on the distal portion 224, such as radiopaque markers that may be provided on or adjacent to the cutting element 220 under fluoroscopy and the like. A desired tension may be applied to or maintained along the apparatus 210 by pulling on its proximal end from the first entry site and/or the distal end from the second entry site (not shown). The electrode 220 may be activated with sufficient electrical energy to ablate and cut the tissue contacted by the electrode 220, and then the apparatus 210 may be directed substantially axially along the first blood vessel, thereby creating a linear incision in the tissue 86 using the cutting element 220. Once a channel of a desired length and size is created, the apparatus 210 may be withdrawn from the patient's body.

Turning to FIGS. 4A-4D, a third preferred embodiment of an apparatus 310 is shown that includes a catheter 312 having a proximal end (not shown), a distal end 316, and defining a longitudinal axis 318. A distal portion 324 of the catheter 312 includes a pair of lateral portions 326a, 326b that are movable between a closed position (FIG. 4A) wherein the lateral portions 326a, 326b are in contact with one another, and an open position (FIG. 4B) wherein the lateral portions 326a, 326b are disposed away from one another, preferably in a generally "Y" shape. Preferably, the lateral portions 326a, 326b each defines a substantially semi-cylindrical cross-section such that the lateral portions 326a, 326b together define a cylindrical profile in the closed position (FIG. 4C) that is similar in cross-section to a proximal portion 314 of the catheter 312 adjacent the distal portion 324. The lateral portions 326a, 326b also preferably define a rounded, substantially atraumatic distal end 316, thereby facilitating insertion and advancement of the catheter 312 within a body lumen, such as a blood vessel.

The lateral portions 326a, 326b may be formed a semi-rigid material, and a hinged or weakened region 328 may be formed where the lateral portions 326a, 326b connect to the proximal portion 314 of the catheter 312, such that the lateral portions 326a, 326b may pivot with respect to one another similar to a clam-shell. For example, the lateral portion 326b may be formed as a separate piece and bonded or otherwise attached to the distal portion 324 at the hinged region 328. The hinged region 328 may be biased towards the closed position to maintain a reduced profile that facilitates insertion of the distal portion into a body lumen. Alternatively, a removable restraint (not shown) may be provided that may secure the lateral portions 326a, 326b together to prevent their inadvertent movement apart from one another. For example, a pullwire and the like may be extended through respective apertures in the lateral portions 326a, 326b or wrapped around the lateral portions 326a, 326b that may be released from the proximal end of the catheter 312 to allow subsequent free movement of the lateral portions 326a, 326b towards the open position. Alternatively, the lateral portions 326a, 326b may be formed from a substantially flexible material, such that they easily deflect away from one another and/or conform to the contour of tortuous anatomy through which the distal portion 324 may be directed.

In addition, each of the lateral portions 326a, 326b preferably includes a lumen 320a, 320b extending from the distal end 316 proximally through the respective lateral portions 326a, 326b. In one form, shown in FIG. 4A, the lumens 320a, 320b extend proximally through the proximal portion 314 of the catheter 312 to its proximal end. Alternatively, as shown in FIG. 4B, one of the lumens 320b' (and optionally both lumens) may terminate at a location proximal to the hinged region 328, i.e., on the distal portion 324 or at an intermediate region of the proximal portion 314. Because of the flexibility or hinging of the lateral portions 326a, 326b, they may be directed away from one another simply by being advanced over respective guidewires, as described further below.

A cutting element 322 is provided between the lateral portions 326 such that the cutting element 322 may be exposed when the lateral portions 326 assume their open position, as best seen in FIGS. 4B and 4D. In a preferred embodiment, the cutting element 322 is a mechanical cutter, such as a blade, that may be coupled to a source of energy, such as an RF generator (not shown), via a conductor 323. Alternatively, the cutting element 322 may be an electrode (not shown), or a mechanical cutter that is not coupled to a source of energy, similar to the previously described embodiments. The cutting element 322 is preferably oriented in a linear configuration extending along a desired cutting plane 340 within which the lateral portions 326 move, corresponding to a desired cutting plane.

An orientation element 332, 333 may be provided on each of the lateral portions 326a, 326b to facilitate imaging and/or rotational orientation of the apparatus 310 during use. Alternatively, the orientation elements 332, 333 may be eliminated, with the guidewires over which the apparatus 310 is introduced providing sufficient guidance and alignment to ensure that the cutting element 322 is aligned along the desired cutting plane.

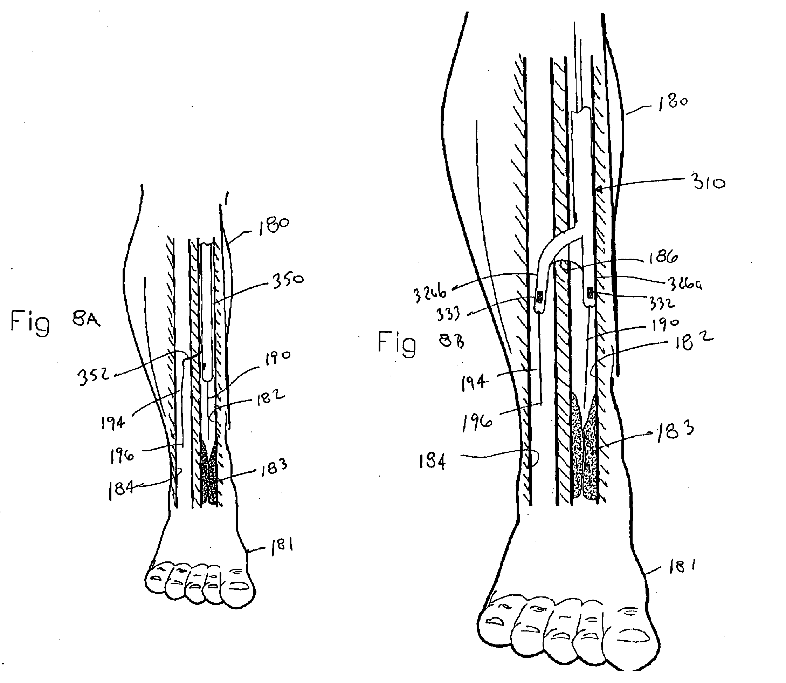

Turning to FIGS. 8A and 8B, the apparatus 310 may be used to create a linear incision or channel between two blood vessels 182, 184, for example, between the tibial artery and a saphenous vein within a leg 180. A channel in the leg 180 may be used to bypass an occlusion 183 within the artery 182, and allow retrograde blood flow through the vein 184 to deliver blood to the foot 181 and/or other locations distal to the occlusion 183.

As shown in FIG. 8A, a first guidewire 190 may be placed within the artery 182 adjacent a crossing site 186. A guidewire delivery device 350 may be advanced over the first guidewire 190 to the crossing site 186, and then a needle 352 on the guidewire delivery device 350 may be directed through the intervening tissue at the crossing site 186 and into the vein 184. A second guidewire 194 may be directed through the needle 352 until its distal end 196 is positioned distally within the vein 184. The guidewire delivery device 350 and needle 352 may then be withdrawn from the patient, leaving the guidewires 190, 194 in place.

Turning to FIG. 8B, the apparatus 310 may then be advanced over the guidewires 190, 194, with each guidewire 190, 194 being directed into a respective lumen 320a, 320b (not shown in FIG. 8B). As the distal portion 324 of the apparatus 310 is advanced to the crossing site 186, the lateral portion 326b follows the second guidewire 194, thereby separating from the other lateral portion 326b, which continues distally past the crossing site 186 over the first guidewire 190. The lateral portion 326b may be advanced through the tissue and the crossing site 186, thereby exposing and positioning the cutting element 322 in contact with the tissue at the crossing site 186. Although the guidewires 190, 194 may provide sufficient alignment to ensure that the cutting element 322 is oriented in a desired cutting plane, i.e., a plane within which the artery 182 and vein 184 lie, fluoroscopy or other imaging may be used to observe the orientation elements 332, 333 to ensure that the apparatus 310 is properly oriented and that the cutting element 322 is exposed and in contact with tissue.

Further distal advancement of the apparatus 310 causes the tissue distal of the crossing site 186 to be sliced by the cutting element 322. When the apparatus 310 has been advanced a predetermined distance, a channel may be created that has a length corresponding substantially to the predetermined distance. If desired, a physical parameter of the patient may be monitored, and the size of the channel adjusted as needed to achieve a desired effect, e.g. flow rate through the channel. The apparatus 310 may be withdrawn proximally, with the lateral portions 326a, 326b returning to the closed position as the lateral portion 326b within the vein 184 returns back into the artery, thereby substantially covering the cutting element 322. The apparatus 310 and the guidewires 190, 194 may then be withdrawn from the patient's body.

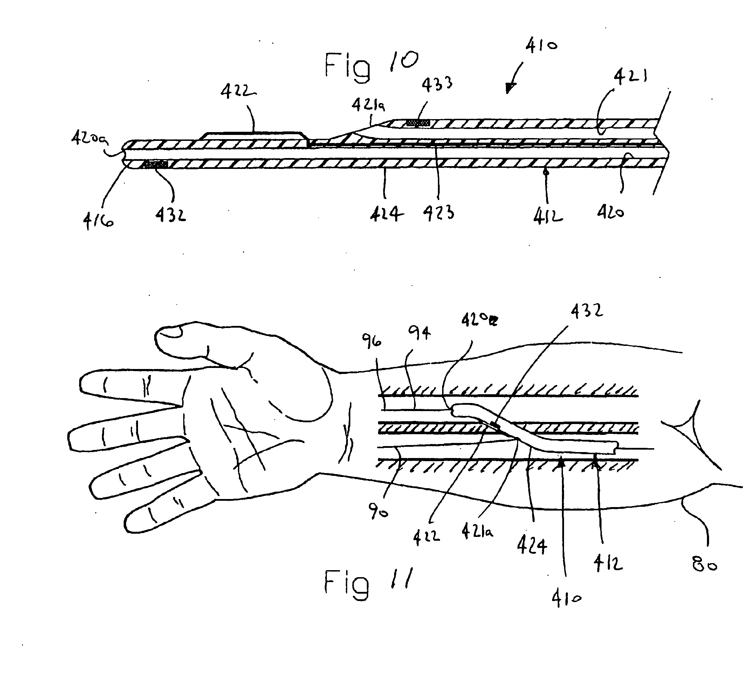

In an alternative embodiment shown in FIG. 10, an apparatus 410 may be provided that includes a catheter 412 including a pair of guidewire lumens 420, 421 that extend from a proximal end (or intermediate region, if rapid exchange is desired) to a distal portion 424 of the catheter 412. The first guidewire lumen 420 extends axially to an outlet port 420a on a distal end of the catheter 412, while the second guidewire lumen 421 has an outlet port 421a at a location proximal to the distal end 416. A cutting element 422 is provided on distal portion 424, preferably between the outlet ports 420a, 421 a, and more preferably axially aligned with the outlet port 421a of the second guidewire lumen 421. One or more orientation markers, such as radiopaque markers 432, 433 may be provided on the distal portion 424, for rotationally orienting the catheter 412, similar to the embodiments described above.

Turning to FIG. 11, the apparatus 410 may be used to create a channel between adjacent blood vessels 82, 84, for example, in a forearm 80. A first guidewire 90 is placed within the first blood vessel 84 adjacent a crossing site 86, and a second guidewire 94 is placed from within the first blood vessel 84 through the tissue at the crossing site 86 and distally into the second blood vessel, similar to the "Y" shaped embodiment described above. The apparatus 410 may then be advanced over the guidewires 90, 94, preferably with the first guidewire 90 within the second guidewire lumen 421, and the second guidewire 94 within the first guidewire lumen 420. As the apparatus 410 is directed to the crossing site 86, the distal portion 424 is guided over the second guidewire 94 into the second blood vessel 82. Because of the location of the outlet port 421 a and the two guidewires 90, 94, the cutting element 422 may be automatically aligned along the desired cutting plane between the two blood vessels 82, 84. Alternatively, the orientation elements 432, 433 may be imaged under fluoroscopy and the like to further rotationally orient the catheter 412, if desired. Thus, the outlet port 421a and the guidewire 90 may cooperate to behave similarly to the hinged lateral portion 326b of the previously described embodiment, i.e., providing a self-aligning function, but without the need for the movable lateral portion, which may result in an apparatus 410 that is easier and/or less expensive to manufacture.

The cutting element 422 may then be activated, and the catheter 412 directed distally, thereby cutting the tissue distal to the crossing site 86 along the desired cutting plane and creating a channel between the blood vessels 82, 84, similar to the previously described embodiments. The guidewires 90, 94 preferably maintain the cutting element 422 in alignment with the desired cutting plane, such that a tissue clip as provided in some of the previous embodiments is not necessary. Alternatively, if desired a tissue clip (not shown) may be provided for providing further stability and/or enhancing contact between the cutting element 422 and the tissue at the crossing site 86. Once a desired channel is created, the apparatus 410 may be withdrawn back into the first blood vessel 84 and out of the patient's body, as may the guidewires 90, 94.

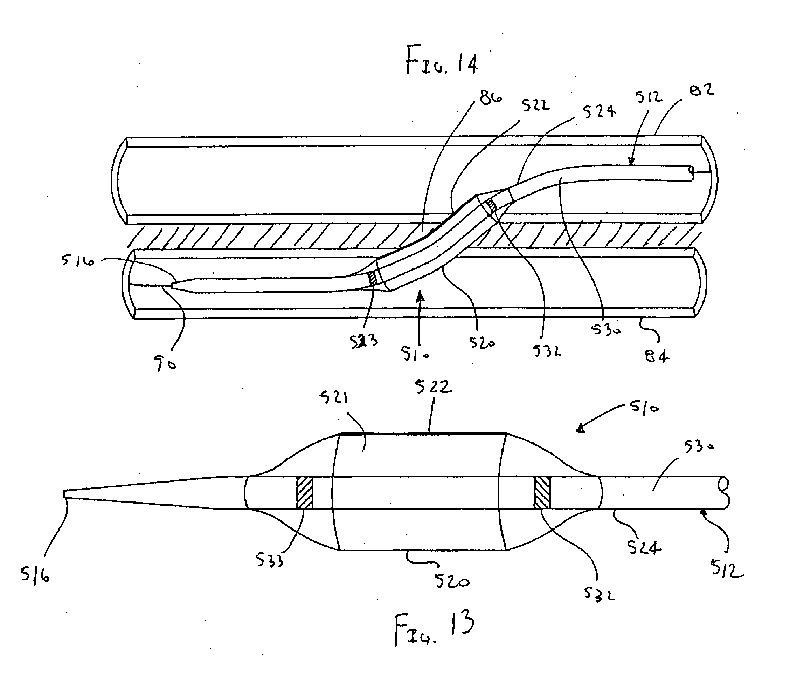

Finally, turning to FIGS. 13 and 14, an apparatus 510 is shown for creating a channel between adjacent blood vessels and the like that includes a catheter 512 having a proximal end (not shown), a distal end 516, and a distal portion 524 that may be inserted into a body lumen, such as blood vessel 82. An expandable member 520 is provided on the distal portion 524, such as an inelastic balloon, that has a cutting element 522 provided along its outer surface 521. Alternatively, mechanical expanders, such as a releasable spring cage, a mechanically actuated lever arm, and the like may be provided instead of a balloon.

Preferably, the cutting element 522 is an electrode that is applied in a linear configuration to the expandable member 520, for example, by adhering electrode material, by vapor deposition, or other known methods for creating an electrically conductive region on a flexible membrane and the like. Alternatively, an outwardly oriented mechanical cutter may also be mounted on the expandable member 520. Orientation elements 532, 533 may be provided on the distal portion 524, for example on or under respective ends of the expandable member 520, to facilitate orienting the distal portion 524, and/or to identify the length or relative position of the cutting element 522. Alternatively, the cutting element 522 itself may be radiopaque or ultrasonically reflective.

With the expandable member 520 in a reduced profile against the outer surface 530 of the catheter 512 (not shown), the distal portion 524 may be advanced over a guidewire 90 previously placed between first and second blood vessels 82, 84 until the expandable member 520 is positioned within the tissue at the crossing site 86. The catheter 512 may be rotationally aligned, for example, under fluoroscopic guidance to orient the cutting element along a desired cutting plane within which the blood vessels 82, 84 lie. The expandable member 520 may then be expanded, as shown in FIG. 14, for example, by introducing an inflation medium, such as saline, therein, until the cutting element 522 substantially contacts the tissue at the crossing site 86.

The cutting element 522 may be activated, for example, energized with RF energy, and the distal portion 524 directed axially to cut the tissue contacted by the cutting element 522. When a channel of sufficient length is created, the cutting element 522 may be deactivated, the expandable member 520 deflated, and the apparatus withdrawn from the patient's body.

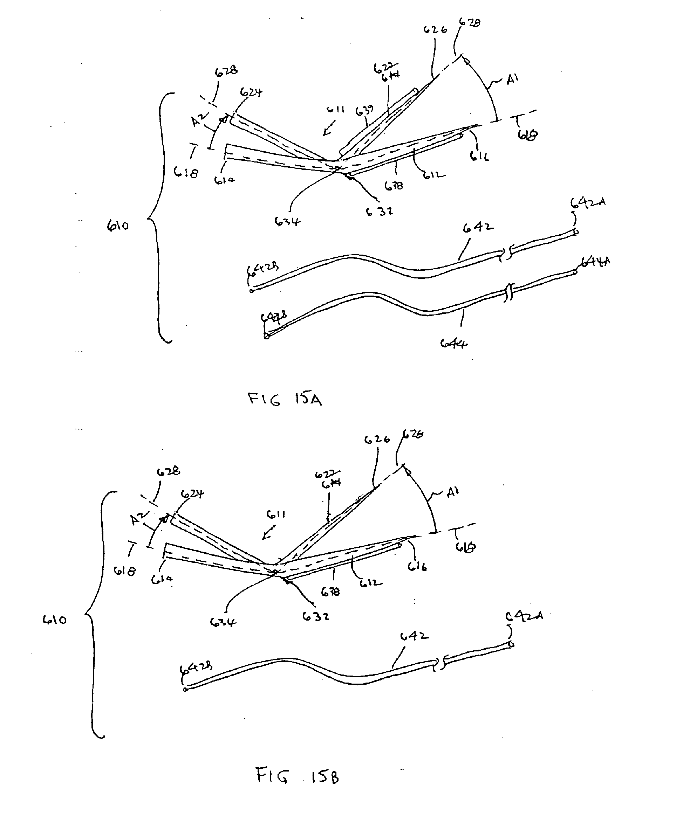

Turning to FIGS. 15A and 15B, exemplary over-the-wire kits 610 are shown, in accordance with another aspect of the present invention. First, FIG. 15A shows a "two-wire" kit 610, including a wire-guided aperture-creating apparatus 611, a first guidewire 642, and a second guidewire 644.

The aperture-creating apparatus 611 has the general form of scissors, including a first elongated element 612 extending between a proximal end 614 and a distal end 616. The first elongated element 612 extends along a first axis 618. The wire-guided aperture-creating apparatus 611 also includes a second elongated element 622 extending between a proximal end 624 and a distal end 626. The second elongated element 622 extends along a second axis 628. The first element 612 is pivotably coupled to the second element 622 by a coupler 632. The first axis 618 substantially intersects the second axis 628 at an intersection point 634. The distal end 616 of the first element 612 is movable with respect to the distal end 626 of the second element 622 about an axis orthogonal to the first and second axes 618, 622 at the intersection point 634. As described below, a tissue cutting assembly 636 is disposed between the first element 612 and the second element 622 elements. In the embodiment of FIG. 15A, the elongated elements 612, 622 are rigid and scissors-like blades extending from point 634 to the distal ends 616, 626 and handles extending from point 634 to proximal ends 618, 628. The blades are dimensioned to fit in the blood vessels of interest, and form the cutting assembly as conventional style scissors blades.

The axes 618 and 628 near the distal ends 616 and 626, form an angle, A1, about an axis which is orthogonal to axes 618, 628 at point 634. The axes 618, 628 near the proximal ends 614, 624 form an angle, A2, about the orthogonal axis at point 634. In this embodiment the handles are angularly offset from the blades (i.e. the bisector of angle Al is angularly offset from the bisector of angle, A2, for each of use of apparatus 611.

Each of the first and second elements 612, 622 includes an associated passage-defining element 638, 639, extending between points 634 and its distal end permitting passage therethrough of one of wires 642, 644.

While in FIG. 15A, the passage-defining elements 638, 639 are shown as discrete cylindrical tubes affixed to an associated one of elongated elements 612, 622; the passage-defining elements 638, 639 may have other forms, for example, channels passing through the interior of elements 612, 622, or as pairs of ring-like guides at opposite ends of the blades of each of elements 612, 622.

The guidewire 642 extends between a distal (or lead) end 642A and a proximal end 642B. Similarly, the guidewire 644 extends between a distal (or lead) end 644A and a proximal end 644B.

FIG. 15B shows a "one-wire" kit 610' similar to the kit 610 of FIG. 15A, although configured for "one-wire" procedures. In FIG. 15B, there are no equivalents to the wire 644 and passage-defining element 639.

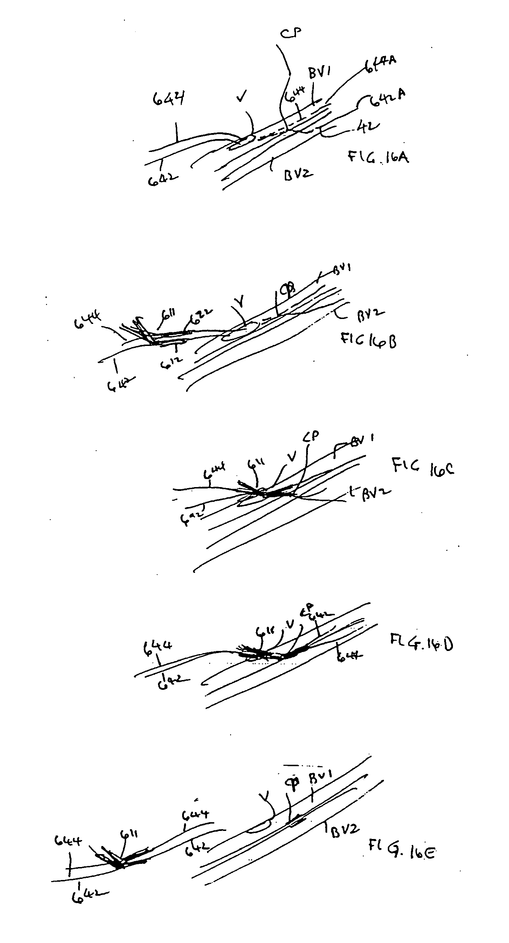

The use of the kit 610 is shown in FIGS. 16A-16E for establishing holes in adjacent sidewalls of two blood vessels BV1 (vein) and BV2 (artery) at an initial cutpoint CP. In an open surgical field, a veinotomy V is made in blood vessel BV1 near the initial cutpoint CP. Then, as shown in FIG. 16A, the guidewire 642 is introduced through veinotomy V and its lead end 642A is advanced toward initial cutpoint CP. When at point CP, the lead end 642A is first driven through the sidewall of BV1 and then the sidewall of BV2 into the lumen of BV2. By way of example, the vessel-to-vessel passage of lead end 642A may be effected using a guidewire 642A having a flexible tip and, optionally, a preformed J-shaped end 642A. The guidewire 642A is preferably inserted within a close-fitting tubular sheath or needle (not shown) so that the preformed J-shape is distorted to match the inner lumen of the sheath until the sheath passes through the initial cutpoint and reaches the lumen of the BV2. At that point, the sheath is maintained relatively stationary and the guidewire 642 is advanced so that, while the guidewire 642 is advanced, the lead end 642A emerges from the sheath. The lead end 642 then reverts to its J-shape, if in place, and advances down the lumen of BV2. Following or before placement of the lead end 642A of the guidewire 642, the lead end 644A of the guidewire 644 is introduced into the veinotomy V and advanced within BV1 to a point beyond the initial cutpoint CP, as shown in FIG. 16A.

As shown in FIG. 15B, the wire-guided apparatus 611 is then positioned over the guidewires 642 and 644 so that the guidewires 642 and 644 are within the passage-defining elements 638 and 639, respectively.

Then, as shown in FIG. 16C, the distal ends of the apparatus 611 are introduced into the veinotomy V. The apparatus 611 is advanced to initial cutpoint CP, where the elongated element 612 tracks the guidewire 642 into BV2, while the elongated element 622 tracks the guidewire 644 and remains in BV 1, as shown in FIG.162D. Then, the surgeon may drive the handles of the apparatus 611 so that the blades close simultaneously, cutting the portions of the sidewalls of BV1 and BV2 between the blades. Following the cut operation, the cutting apparatus 611 and guidewires 642, 644 are withdrawn from BV1 and BV2 as shown in FIG. 16E. A bridging connection may then be established between BV1 and BV2, for example, using couplers, such as those disclosed in U.S. Patent No. 5,830,222, which is expressly incorporated herein by reference.

In a "one-wire" procedure, the kit 610 of FIG. 15B may be used in a similar manner to the method described above, except that the elongated element 622 merely remains in BV1 without tracking a guidewire.

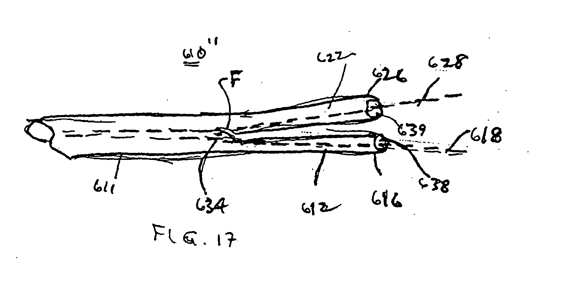

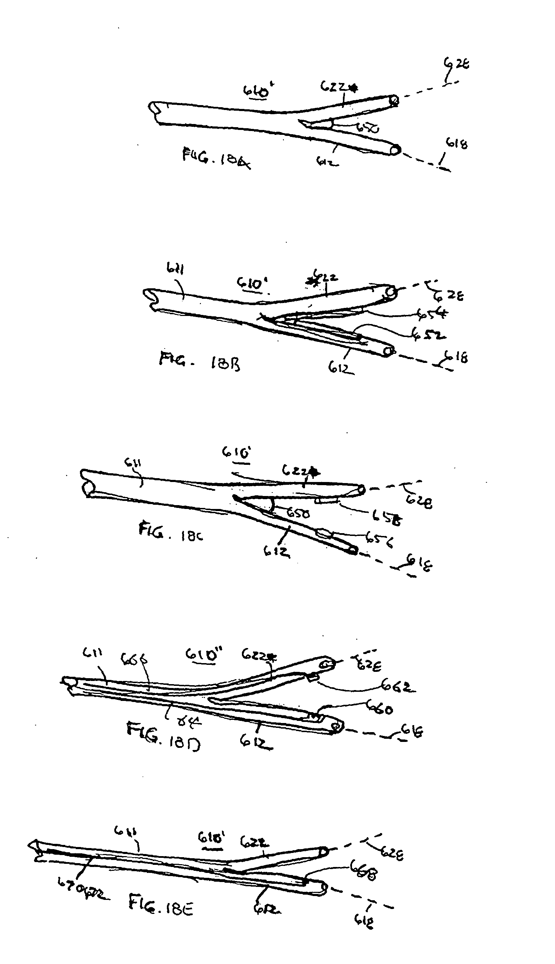

While the wire-guided aperture-creating apparatus described above has a rigid scissors-like form, a wire-guided aperture-creating apparatus in accordance with the present invention may take alternate forms. For example, FIG. 17 shows an alternative embodiment of a aperture-creating apparatus 610" that includes a flexible catheter 611 having a bifurcation at its distal end defining elongated elements 612, 622 having distal ends 616, 626, respectively, and extending along axes 626, 628, respectively. The catheter 611 has a flexure F at the junction 634 of the bifurcated portions so that the distal ends 616, 626 are moveable with respect to each other about an axis orthogonal to axes 618, 628 at point 634. As illustrated in FIG. 17, each of the distal ends 616, 626 includes a respective passage-defining aperture 638, 639, each being suitable for tracking over a respective one of guidewires 642, 644. In "one-wire" kits, the aperture 639 may not be present. In alternative forms of the embodiment of FIG. 17, the bifurcated ends may be flexible, or relatively stiff or rigid. In addition, the tissue aperture-creating assembly may take many forms. Some such forms are shown schematically in FIGS. 18A-18E. In FIG. 18A, an outwardly, facing blade 650 is disposed at the junction of the bifurcated portions. With this configuration, mere advancement of the apparatus 610" will effect cutting once the distal ends 616, 626 are within the respective vessels BV1 and BV2. FIG. 18B shows an apparatus 610" having an elongated blade 652 on element 612 and an opposing anvil 654 on element 624. Similarly, FIG. 18C shows an apparatus 610" having a hole punch 656 on element 612 and an anvil 658 on element 624, together with a blade 650 at the junction of the bifurcated portions. This configuration is useful for cutting an initial hole at the point CP and then a second hole separated from the initial hole by the distance of the hole punch 656 from the blade 650. FIG. 18D shows an apparatus 611 having an RF coil 660 on element 612, and a grounded element 662 on element 622, where the coil 660 and ground element 662 are electrically coupled by wires 664, 666 to an external RF generator. FIG. 18E shows a laser cutter 668 on element 612 which may be selectively actuated via wires 670, 672, to ablate tissue adjacent thereto.

All of the above catheter configurations have an advantage over the rigid scissors-like configuration of FIGS. 15A and 15B, i.e., that the veinotomy may be relatively remote from the initial cutpoint (since the catheter may be of any desire length). In contrast, the scissors-like configurations of Figs. 15A and 15B require externally applied forces on the handles to effect the cutting, and those forces must be applied relatively close to the initial cutpoint. In other forms, however, the scissors-like elements 612, 622 may be remotely actuated by a conventional mechanical (or electrically driven) linkage.

The embodiment of FIG. 18A is particularly easy to use since mere advancement of the apparatus 610" effects cutting (via blade 650). The configurations of FIGS. 18B-18E require remote driving of the distal ends 616, 626 toward each other, although this may be effected by using sheathed wires, as in the sidewall piercing device described above, or other conventional selectively actuatable closing assemblies.

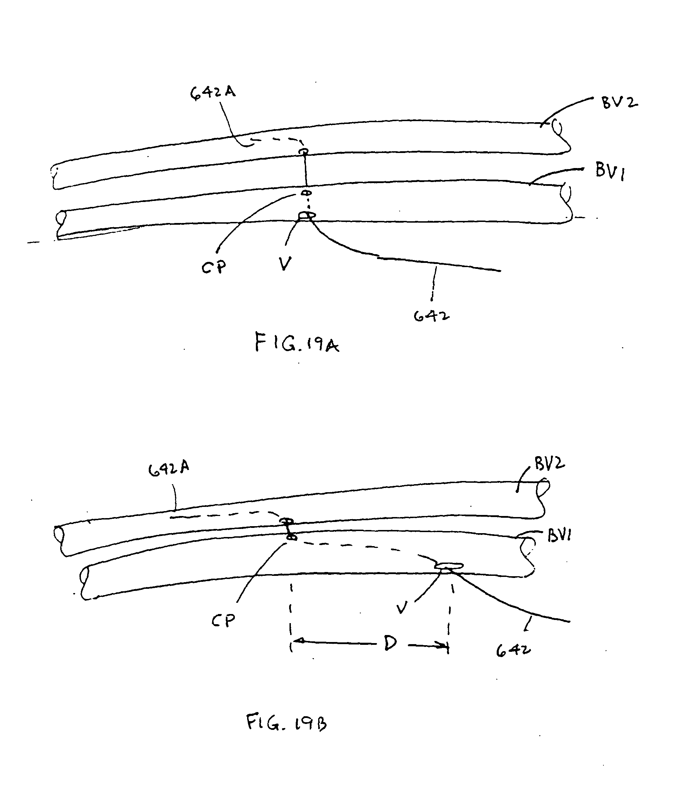

The embodiments of FIGS. 17-18E may be used with a guidewire 642 introduced at an access point V at or near or opposite to the initial cutpoint CP (as shown in FIG. 19A) or with a guidewire 642 introduced at a distance D from that initial cutpoint CP (as shown in FIG. 19B).

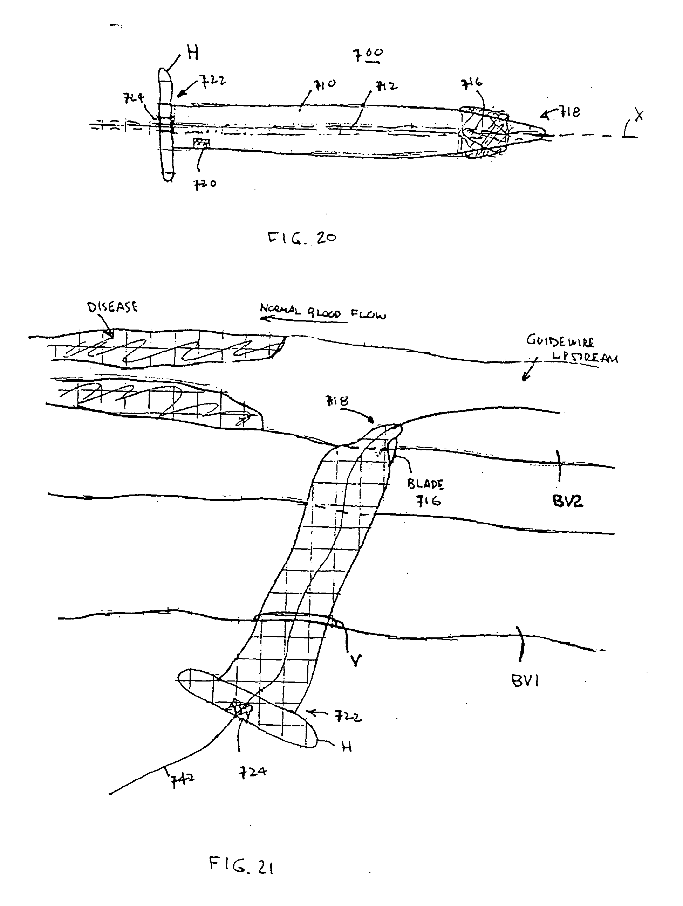

Additional aperture-creating apparatus adapted for following a single guidewire (in the configurations of FIGS. 19A and 19B) are shown in FIGS. 20-27B. For example, FIG. 20 shows an aperture-creating apparatus 700 that includes an elongated element 710 having a central lumen 712 (for passage over a guidewire 742). The element 710 may be rigid or flexible, and includes a tissue cutting, tissue removing, tissue ablating or tissue enlarging element 716 at or near its distal tip 718. An optional angular orientation (about an axis of lumen 712) indicator 720 is shown near the proximal end 722 of element 710. A hemostasis valve 724 may optionally be positioned (e.g., near proximal end 722) on element 710 to block exit blood flow through the lumen 712 while allowing passage of the guidewire 742, if desired. FIG. 21 shows the aperture-creating apparatus 700 in use to establish a flowpath from a blood vessel upstream of a diseased segment to an adjacent vessel. In use, a suitable coupler or scaffold (not shown) may be used to couple the apertures established by a blade B in aperture-creating apparatus 700, so that a well-defined blood flowpath is established between the two vessels.

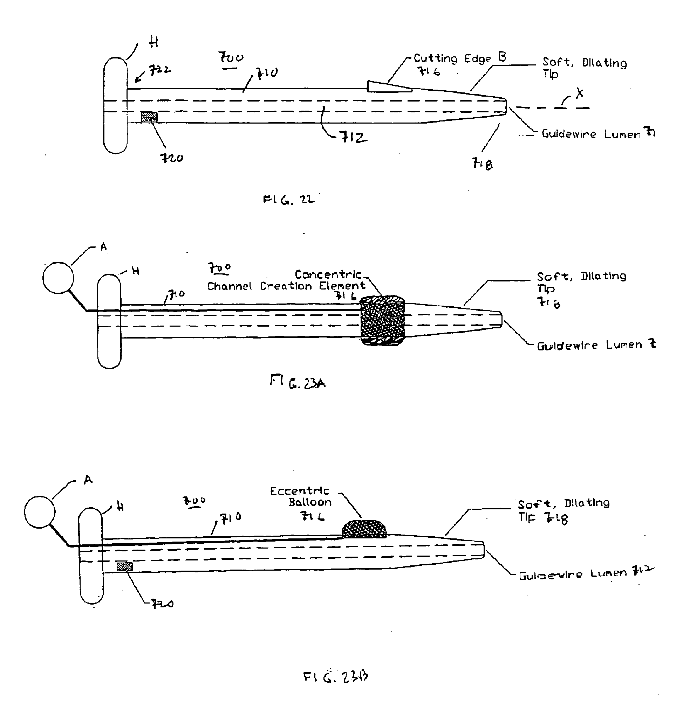

FIG. 22 shows an exemplary form for the aperture-creating apparatus 700 having a rigid element 710 with a blade B near its distal tip 718 and a handle H at the proximal end 722. In this illustrated form, the distal end of element 710 is relatively soft or flexible. An orientation marker 720 is positioned near the proximal end 722.

FIGS. 23A and 23B show similar exemplary forms of aperture-creating apparatus 700 but where the tissue enlarging elements are in the form of remotely a deployable/inflatable concentric balloon 116 (FIG. 23A) and an eccentric balloon 716 (FIG 923). In the latter form, the orientation marker 720 is preferably employed so that in use the handle H may be used to rotate the element 710 and the resultant motion of the eccentric balloon is known. In FIGS. 23A and 23B, an actuator A is shown for selectively inflating the balloons.

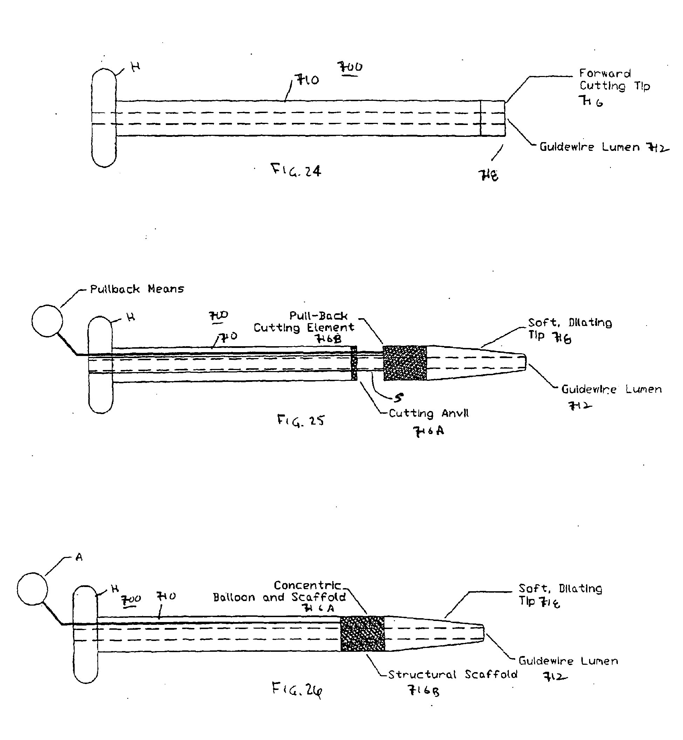

FIG. 24 shows an aperture-creating apparatus 700 with an elongated element 710 with a central lumen 712 and having a handle H at its proximal end and a circular cutting edge 716 at its distal end 718. This apparatus 700 operates in a "push forward" mode where the user applies a force to the handle H to direct the cutting edge 716 against the vessel sidewalls.

FIG. 25 shows an aperture-creating apparatus 700 with a cylindrical shell 710 slidably disposed over a shaft S that has an inner guidewire lumen 712. The leading edge 716A forms an anvil against which a cutting edge 716B extending from the distal end of the shaft S may be driven in response to a pull-back force applied by a user.

FIG. 26 shows another aperture-creating apparatus 700 that includes a shaft 710 (including a guidewire lumen 712) with a concentric balloon 716A and optional scaffold 716B near its distal end 718. A remote actuator A is used to selectively inflate the balloon 716A. In operation, the balloon 716A may be inflated to create desired apertures. Optionally, the balloon 716A may include a scaffold member 716B disposed thereon (e.g., a reinforced balloon) to aid in aperture creation. In some cases, it may be desirable to permanently implant the scaffold member 716B to further aid in creating a fluid path between the apertures.

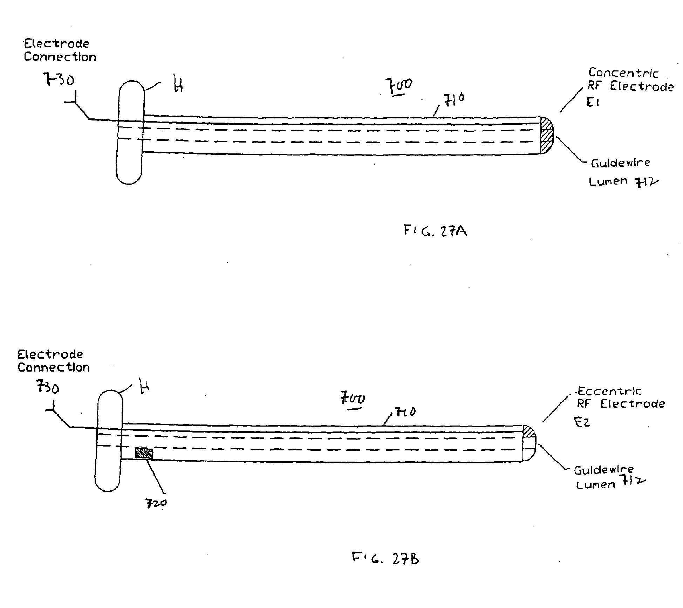

FIGS. 27A and 27B show forms of aperture-creating apparatus 700 wherein the aperture-creating element is either an concentric ablation electrode E1 (FIG. 27A) or an eccentric ablation electrode E2 (FIG. 27B). The electrodes E1, E2 may be selectively activated by way of electrode connections 730. An orientation marker 724 may be used with the eccentric electrode to allow the operator to direct the cutting energy.

If the element 710 of the aperture-creating apparatus 700 in FIGS. 20-27B is substantially rigid, the guidewire deployments of FIG. 19A are preferably used. If the element 710 is substantially flexible, the guidewire deployments of either of FIGS. 19A and 19B may be used.

While the invention is susceptible to various modifications, and alternative forms, specific examples thereof have been shown in the drawings and are herein described in detail. It should be understood, however, that the invention is not to be limited to the particular forms disclosed, butis limited by the scope of the appended claims.

## Claims

1. A wire-guided cutting assembly (10, 110, 310, 611, 610") for simultaneously cutting a slit in adjacent portions of a first blood vessel and a second blood vessel during a surgical procedure, comprising:
a first elongated element (12, 112, 326a, 612) extending along a first axis (18, 118, 618) between a proximal end (14, 114, 614) and a distal end (16, 116, 616);
a second elongated element (34, 134, 326b, 622) extending along a second axis (628) between a proximal end (624) and a distal end (626), the second elongated element (34, 134, 326b, 622) being pivotably coupled to the first elongated element (12, 112, 326a, 612); and
a tissue cutting assembly (22, 122, 322) disposed between the first and second elongated elements (12, 34, 112, 134, 326a, 326b, 612, 622);
wherein at least one of the first and second elongated elements (12, 34, 112, 134, 326a, 326b, 612, 622) includes an interior passage-defining element (638, 639) permitting passage therethrough of a guidewire (190, 194, 642, 644).

2. The wire-guided cutting assembly (611) according to claim 1, further comprising:
a coupler (632) pivotably coupling the first elongated element (612) to the second elongated element (622), the first axis (618) substantially intersecting the second axis (628), the distal end (616) of the first elongated element (612) being movable with respect to the distal end (626) of the second elongated element (622) about an axis orthogonal to the first and second axes (618, 628).

3. The wire-guided cutting assembly (10, 110, 310, 611, 610") according to claim 1 or claim 2, wherein each of the first and second elongated elements (12, 34, 112, 134, 326a, 326b, 612, 622) includes an interior passage-defining element (638, 639) permitting passage therethrough of an associated one of a first guidewire (190, 642) and a second guidewire (194, 644).

4. The wire-guided cutting assembly (611) according to one of claims 2 to 3, wherein the first and second elongated elements (612, 622) are substantially rigid and the first and second elongated elements (612, 622) are connected at a pivotal coupling point pivoting about an axis orthogonal to the first and second axes.

5. The wire-guided cutting assembly (611) according to claim 4, wherein the wire-guided cutting assembly (611) comprises opposing cutting elements extending in a first direction from an intermediate point, and a handle cutting control element extending from the intermediate point in a direction opposite to the first direction, the pivotal coupling being at the intermediate point.

6. The wire-guided cutting assembly (611) according to claim 5, wherein a bisector of an angle (A1) formed by the first and second axes of the opposed cutting elements is angularly offset with respect to a bisector of an angle (A2) formed by the first and second axes of the handle cutting control element.

7. The wire-guided cutting assembly (10, 110, 310, 610'') according to one of claims 2 to 3, wherein at least one of the first and second elongated elements (12, 34, 112, 134, 326a, 326b, 612, 622) is flexible at least near their distal ends (16, 116, 616, 626).

8. The wire-guided cutting assembly (10, 110, 310, 610") according to one of claims 2 to 3, wherein both of the first and second elongated elements (12, 34, 112, 134, 326a, 326b, 612, 622) are flexible at least near their distal ends (16, 116, 616, 626).

9. The wire-guided cutting assembly (310, 610") according to one of claims 2, 3, 7 and 8, the tissue cutting assembly (322) comprising a blade (650) at the pivotal coupling point, the blade (650) facing away from the pivotal coupling point.

10. The wire-guided cutting assembly (310, 610") according to claim 2, wherein the tissue cutting assembly (322) comprises a cooperating blade (652) and anvil (654) on the first and second elongated elements (326a, 326b, 612, 622).

11. The wire-guided cutting assembly (310, 610") according to claim 2, wherein the tissue cutting assembly (322) comprises a cooperating hole punch (656) and anvil (658) on the first and second elongated elements (326a, 326b, 612, 622).

12. The wire-guided cutting assembly (310, 610") according to claim 1 or claim 2, wherein the tissue cutting assembly (322) comprises an RF cutter (660, 662).

13. The wire-guided cutting assembly (310, 610") according to claim 1 or claim 2, wherein the tissue cutting assembly (322) comprises a laser cutter (668).

14. The wire-guided cutting assembly (310, 610") according to claim 1 or claim 2, wherein the tissue cutting assembly (322) comprises a heat cutter.

15. The wire-guided cutting assembly (310, 610") according to any of claims 1, 3 and 7-9, further comprising a catheter (312, 611) a distal portion (324) of which comprises the first elongated element (326a, 612) and the second elongated element (326b, 622).

16. The wire-guided cutting assembly (310) according to claim 15, further comprising an orientation element (332, 333) provided on each of the first elongated element (326a) and the second elongated element (326b) to facilitate imaging and/or rotational orientation of the wire-guided cutting assembly (310) during use.

17. The wire-guided cutting assembly (10, 110) according to claim 1, the first elongated element (12, 112) being a catheter, the tissue cutting assembly (22, 122) being a. cutting element fixed at a predetermined location on a periphery of a distal portion (24, 124) of the catheter, the cutting element configured for creating a linear incision in a wall of a body lumen substantially parallel to a longitudinal axis of the catheter, and the second elongated element (34, 134) being a clip adjacent to the cutting element for engaging tissue between the clip and an outer surface of the catheter for maintaining the cutting element in contact with the tissue.

18. The wire-guided cutting assembly (10) according to claim 17, further comprising a sheath (40) for selectively covering and uncovering the clip, the sheath (40) maintaining the clip in close contact with the outer surface of the catheter when the sheath (40) covers the flexible clip.

19. The wire-guided cutting assembly (10, 110) of claim 17, wherein the clip comprises a flexible portion for biasing a loose end (38, 138) of the clip away from the outer surface of the first elongate member (12, 112) to facilitate engaging tissue between the clip and the outer surface of the first elongate member (12, 112).

20. The wire-guided cutting assembly (10, 110) of claim 17, further comprising a filament (140) releasably connected to a loose end (38, 138) of the clip, the filament (140) extending proximally to the proximal end of the first elongate member (12, 112), whereby the filament (140) may hold the loose end (38, 138) of the clip in close contact with the outer surface of the first elongate member (12, 112) until the filament (140) is released from the proximal end (14, 114) of the first elongate member (12, 112).

21. The wire-guided cutting assembly (10) of claim 17, wherein the clip is at least partially retractable into the first elongate member (12).

22. The wire-guided cutting assembly (10, 110) of claim 17, further comprising an orientation element (33, 133) on the clip for monitoring its position relative to the cutting element.

23. An apparatus (410) for creating a channel between adjacent blood vessels (82, 84), comprising:
a catheter (412) including a first guidewire lumen (420) and a second guidewire lumen (421) that extend from a proximal end or an intermediate region of the catheter (412) to a distal portion (424) of the catheter (412),
the first guidewire lumen (420) extending axially to a first outlet port (420a) on a distal end (416) of the catheter (412),
the second guidewire lumen (421) having a second outlet port (421a) at a location proximal to the distal end (416) of the catheter (412),
and a cutting element (422) provided on the distal portion (424) of the catheter (412) between the first and the second outlet port (420a, 421a).

## Patentansprüche

1. Drahtgeführte Schneide-Anordnung (10, 110, 310, 611, 610") zum gleichzeitigen Schneiden eines Schlitzes in benachbarten Abschnitten eines ersten Blutgefäßes und eines zweiten Blutgefäßes während eines chirurgischen Eingriffs, aufweisend:
ein erstes längliches Element (12, 112, 326a, 612), das sich entlang einer ersten Achse (18, 118, 618) zwischen einem proximalen Ende (14, 114, 614) und einem distalen Ende (16, 116, 616) erstreckt,
ein zweites längliches Element (34, 134, 326b, 622), das sich entlang einer zweiten Achse (628) zwischen einem proximalen Ende (624) und einem distalen Ende (626) erstreckt, wobei das zweite längliche Element (34, 134, 326b, 622) schwenkbar mit dem ersten länglichen Element (12, 112, 326a, 612) verbunden ist, und
eine Gewebe-Schneide-Anordnung (22, 122, 322), die zwischen dem ersten und dem zweiten länglichen Element (12, 34, 112, 134, 326a, 326b, 612, 622) angeordnet ist,
wobei mindestens eines von dem ersten und dem zweiten länglichen Element (12, 34, 112, 134, 326a, 326b, 612, 622) ein Innendurchgangs-Definitionselement (638, 639) aufweist, das ein Passieren eines Führungsdrahtes (190, 194, 642, 644) dort hindurch ermöglicht.

2. Drahtgeführte Schneide-Anordnung (611) gemäß Anspruch 1, ferner aufweisend:
ein Verbindungsstück (632), welches das erste längliche Element (612) schwenkbar mit dem zweiten länglichen Element (622) verbindet, wobei die erste Achse (618) sich im Wesentlichen mit der zweiten Achse (628) überschneidet, wobei das distale Ende (616) des ersten länglichen Elements (612) relativ zu dem distalen Ende (626) des zweiten länglichen Elements (622) um eine Achse orthogonal zu der ersten und der zweiten Achse (618, 628) bewegbar ist.

3. Drahtgeführte Schneide-Anordnung (10, 110, 310, 611, 610") gemäß Anspruch 1 oder Anspruch 2, wobei jedes von dem ersten und dem zweiten länglichen Element (12, 34, 112, 134, 326a, 326b, 612, 622) ein Innendurchgangs-Definitionselement (638, 639) aufweist, das ein Passieren eines assoziierten von einem ersten Führungsdraht (190, 642) und einem zweiten Führungsdraht (194, 644) dort hindurch ermöglicht.

4. Drahtgeführte Schneide-Anordnung (611) gemäß einem der Ansprüche 2 bis 3, wobei das erste und das zweite längliche Element (612, 622) im Wesentlichen steif sind, und wobei das erste und das zweite längliche Element (612, 622) an einem Schwenkverbindungspunkt schwenkbar um eine zu der ersten und der zweiten Achse orthogonale Achse verbunden sind.

5. Drahtgeführte Schneide-Anordnung (611) gemäß Anspruch 4, wobei die drahtgeführte Schneide-Anordnung (611) aufweist: gegenüberliegende Schneide-Elemente, die sich in einer ersten Richtung von einem dazwischenliegenden Punkt erstrecken, und ein Handgriff-Schneide-SteuerElement, das sich von dem dazwischenliegenden Punkt in einer Richtung entgegengesetzt zu der ersten Richtung erstreckt, wobei die Schwenkverbindung an dem dazwischenliegenden Punkt liegt.

6. Drahtgeführte Schneide-Anordnung (611) gemäß Anspruch 5, wobei eine Winkelhalbierende eines Winkels (A1), der von der ersten und der zweiten Achse der gegenüberliegenden Schneide-Elemente gebildet wird, relativ zu einer Winkelhalbierenden eines Winkels (A2), der von der ersten und der zweiten Achse des Handgriff-Schneide-SteuerElements gebildet wird, winklig versetzt ist.

7. Drahtgeführte Schneide-Anordnung (10, 110, 310, 610") gemäß einem der Ansprüche 2 bis 3, wobei mindestens eines von dem ersten und dem zweiten länglichen Element (12, 34, 112, 134, 326a, 326b, 612, 622) zumindest nahe bei ihren distalen Enden (16, 116, 616, 626) flexibel ist.

8. Drahtgeführte Schneide-Anordnung (10, 110, 310, 610") gemäß einem der Ansprüche 2 bis 3, wobei beide von dem ersten und dem zweiten länglichen Element (12, 34, 112, 134, 326a, 326b, 612, 622) zumindest nahe bei ihren distalen Enden (16, 116, 616, 626) flexibel sind.

9. Drahtgeführte Schneide-Anordnung (310, 610") gemäß einem der Ansprüche 2, 3, 7 und 8, wobei die Gewebe-Schneide-Anordnung (322) an dem Schwenkverbindungspunkt eine Schneide (650) aufweist, wobei die Schneide (650) von dem Schwenkverbindungspunkt abgewandt ist.

10. Drahtgeführte Schneide-Anordnung (310, 610") gemäß Anspruch 2, wobei die Gewebe-Schneide-Anordnung (322) eine zusammenwirkende Schneide (652) und Amboss (654) an dem ersten und zweiten länglichen Element (326a, 326b, 612, 622) aufweist.

11. Drahtgeführte Schneide-Anordnung (310, 610") gemäß Anspruch 2, wobei die Gewebe-Schneide-Anordnung (322) eine zusammenwirkende Lochstanze (656) und Amboss (658) an dem ersten und dem zweiten länglichen Element (326a, 326b, 612, 622) aufweist.

12. Drahtgeführte Schneide-Anordnung (319, 610") gemäß Anspruch 1 oder 2, wobei die Gewebe-Schneide-Anordnung (322) einen Radiofrequenz-Schneider (660, 662) aufweist.

13. Drahtgeführte Schneide-Anordnung (310, 610") gemäß Anspruch 1 oder Anspruch 2, wobei die Gewebe-Schneide-Anordnung (322) einen Laser-Schneider (668) aufweist.

14. Drahtgeführte Schneide-Anordnung (310, 610") gemäß Anspruch 1 oder Anspruch 2, wobei die Gewebe-Schneide-Anordnung (322) einen Wärmeschneider aufweist.

15. Drahtgeführte Schneide-Anordnung (310, 610") gemäß einem der Ansprüche 1, 3 und 7 bis 9, ferner einen Katheter (312, 611) aufweisend, wobei ein distaler Abschnitt (324) desselben das erste längliche Element (326a, 612) und das zweite längliche Element (326b, 622) aufweist.

16. Drahtgeführte Schneide-Anordnung (310) gemäß Anspruch 15, ferner ein Ausrichtungselement (332, 333) aufweisend, das an jedem von dem ersten länglichen Element (326a) und dem zweiten länglichen Element (326b) vorgesehen ist, um eine Bildgebung und/oder Dreh-Ausrichtung der drahtgeführten Schneide-Anordnung (310) während der Verwendung zu erleichtern.

17. Drahtgeführte Schneide-Anordnung (10, 110) gemäß Anspruch 1, wobei das erste längliche Element (12, 112) ein Katheter ist, wobei die Gewebe-Schneide-Anordnung (22, 122) ein Schneide-Element ist, das an einer vorbestimmten Position an einem Umfang eines distalen Abschnitts (24, 124) des Katheters angebracht ist, wobei das Schneide-Element zum Erzeugen eines linearen Schnitts in einer Wand eines Körper-Lumens im Wesentlichen parallel zu einer Längsachse des Katheters eingerichtet ist, und wobei das zweite längliche Element (34, 134) eine Klemme benachbart zu dem Schneide-Element ist, für einen Eingriff mit Gewebe zwischen der Klemme und einer Außenfläche des Katheters zum Halten des Schneide-Elements in Kontakt mit dem Gewebe.

18. Drahtgeführte Schneide-Anordnung (10) gemäß Anspruch 17, ferner eine Hülle (40) zum selektiven Bedecken und Freilegen der Klemme aufweisend, wobei die Hülle (40) die Klemme in engem Kontakt mit der Außenfläche des Katheters hält, wenn die Hülle (40) die flexible Klemme bedeckt.

19. Drahtgeführte Schneide-Anordnung (10, 110) gemäß Anspruch 17, wobei die Klemme einen flexiblen Abschnitt zum Vorspannen eines losen Endes (38, 138) der Klemme weg von der Außenfläche des ersten länglichen Elements (12, 112) aufweist, um einen Eingriff mit Gewebe zwischen der Klemme und der Außenfläche des ersten länglichen Elements (12, 112) zu erleichtern.

20. Drahtgeführte Schneide-Anordnung (10, 110) gemäß Anspruch 17, ferner ein Filament (140) aufweisend, das lösbar mit einem losen Ende (38, 138) der Klemme verbunden ist, wobei sich das Filament (140) proximal zu dem proximalen Ende des ersten länglichen Elements (12, 112) erstreckt, wodurch das Filament (140) das lose Ende (38, 138) der Klemme in engem Kontakt zu der Außenfläche des ersten länglichen Elements (12, 112) halten kann, bis das Filament (140) von dem proximalen Ende (14, 114) des ersten länglichen Elements (12, 112) gelöst ist.

21. Drahtgeführte Schneide-Anordnung (10) gemäß Anspruch 17, wobei die Klemme zumindest teilweise in das erste längliche Element (12) zurückziehbar ist.

22. Drahtgeführte Schneide-Anordnung (10, 110) gemäß Anspruch 17, ferner ein Ausrichtungs-Element (33, 133) an der Klemme aufweisend, um ihre Position relativ zu dem Schneide-Element zu überwachen.

23. Vorrichtung (410) zum Erzeugen eines Kanals zwischen benachbarten Blutgefäßen (82, 84), aufweisend:
einen Katheter (412), der ein erstes Führungsdraht-Lumen (420) und ein zweites Führungsdraht-Lumen (421) aufweist, die sich von einem proximalen Ende oder einem dazwischenliegenden Bereich des Katheters (412) zu einem distalen Abschnitt (424) des Katheters (412) erstrecken,
wobei sich das erste Führungsdraht-Lumen (420) axial zu dem ersten Auslass-Anschluss (420a) an einem distalen Ende (416) des Katheters (412) erstreckt,
wobei das zweite Führungsdraht-Lumen (421) einen zweiten Auslass-Anschluss (421a) an einer Position proximal zu dem distalen Ende (416) des Katheters (412) aufweist,
und ein Schneide-Element (422), das an dem distalen Abschnitt (424) des Katheters (412) zwischen dem ersten und dem zweiten Auslass-Anschluss (420a, 421a) vorgesehen ist.

## Revendications

1. Ensemble de coupe (10, 110, 310, 611, 610") guidé par un fil, permettant de couper simultanément une fente dans des parties adjacentes d'un premier vaisseau sanguin et d'un deuxième vaisseau sanguin pendant une procédure chirurgicale, comprenant :
un premier élément allongé (12, 112, 326a, 612) s'étendant le long d'un premier axe (18, 118, 618), entre une extrémité proximale (14, 114, 614) et une extrémité distale (16, 116, 616) ;
un deuxième élément allongé (34, 134, 326b, 622) s'étendant le long d'un deuxième axe (628), entre une extrémité proximale (624) et une extrémité distale (626), le deuxième élément allongé (34, 134, 326b, 622) étant couplé à pivot au premier élément allongé (12, 112, 326a, 612) ; et
un ensemble (22, 122, 322) de coupe de tissu, disposé entre les premier et deuxième éléments allongés (12, 34, 112, 134, 326a, 326b, 612, 622) ;
au moins un des premier et deuxième éléments allongés (12, 34, 112, 134, 326a, 326b, 612, 622) comprenant un élément définissant un passage intérieur (638, 639) permettant le passage d'un fil de guidage (190, 194, 642, 644) à travers lui.

2. Ensemble de coupe (611) guidé par un fil selon la revendication 1, comprenant en outre :
un coupleur (632) couplant à pivot le premier élément allongé (612) au deuxième élément allongé (622), le premier axe (618) coupant pratiquement le deuxième axe (628), l'extrémité distale (616) du premier élément allongé (612) étant mobile par rapport à l'extrémité distale (626) du deuxième élément allongé (622), autour d'un axe orthogonal aux premier et deuxième axes (618, 628).

3. Ensemble de coupe (10, 110, 310, 611, 610") guidé par un fil selon la revendication 1 ou la revendication 2, dans lequel chacun des premier et deuxième éléments allongés (12, 34, 112, 134, 326a, 326b, 612, 622) comprend un élément définissant un passage intérieur (638, 639) permettant le passage d'un premier fil de guidage (190, 642) et d'un second fil de guidage (194, 644) qui lui est associé à travers lui.

4. Ensemble de coupe (611) guidé par un fil selon l'une des revendications 2 à 3, dans lequel les premier et deuxième éléments allongés (612, 622) sont sensiblement rigides et où les premier et deuxième éléments allongés (612, 622) sont raccordés à un point de couplage pivotant qui pivote autour d'un axe orthogonal aux premier et deuxième axes.

5. Ensemble de coupe (611) guidé par un fil selon la revendication 4, dans lequel l'ensemble de coupe (611) guidé par un fil comprend des éléments coupants opposés qui s'étendent selon une première direction à partir d'un point intermédiaire, et un élément de commande de coupe à poignée, qui s'étend à partir du point intermédiaire dans une direction opposée à la première direction, le couplage pivotant se trouvant au point intermédiaire.

6. Ensemble de coupe (611) guidé par un fil selon la revendication 5, dans lequel une bissectrice d'un angle (A1) formée par les premier et deuxième axes des éléments opposés de coupe est décalé angulairement par rapport à une bissectrice d'un angle (A2) formée par les premier et deuxième axes de l'élément de commande de coupe à poignée.

7. Ensemble de coupe (10, 110, 310, 610") guidé par un fil selon l'une des revendications 2 à 3, dans lequel au moins un des premier et deuxième éléments allongés (12, 34, 112, 134, 326a, 326b, 612, 622) est flexible, au moins à proximité de leurs extrémités distales (16, 116, 616, 626).

8. Ensemble de coupe (10, 110, 310, 610") guidé par un fil selon l'une des revendications 2 à 3, dans lequel le premier et le deuxième éléments allongés (12, 34, 112, 134, 326a, 326b, 612, 622) sont tous deux flexibles, au moins à proximité de leurs extrémités distales (16, 116, 616, 626).

9. Ensemble de coupe (310, 610") guidé par un fil selon l'une des revendications 2, 3, 7 et 8, l'ensemble (322) de coupe de tissu comprenant une lame (650) au niveau du point de couplage pivotant, la lame (650) étant dirigée à l'opposé du point de couplage pivotant.

10. Ensemble de coupe (310, 610") guidé par un fil selon la revendication 2, dans lequel l'ensemble (322) de coupe de tissu comprend une lame coopérante (652) et une enclume (654) sur les premier et deuxième éléments allongés (326a, 326b, 612,622).

11. Ensemble de coupe (310, 610") guidé par un fil selon la revendication 2, dans lequel l'ensemble (322) de coupe de tissu comprend un poinçon perforant coopérant (656) et une enclume (658) sur les premier et deuxième éléments allongés (326a, 326b, 612, 622).

12. Ensemble de coupe (310, 610") guidé par un fil selon la revendication 1 ou la revendication 2, dans lequel l'ensemble (322) de coupe de tissu comprend un coupeur HF (660, 662).

13. Ensemble de coupe (310, 610") guidé par un fil selon la revendication 1 ou la revendication 2, dans lequel l'ensemble (322) de coupe de tissu comprend un coupeur au laser (668).

14. Ensemble de coupe (310, 610") guidé par un fil selon la revendication 1 ou la revendication 2, dans lequel l'ensemble (322) de coupe de tissu comprend un coupeur thermique.

15. Ensemble de coupe (310, 610") guidé par un fil selon l'une quelconque des revendications 1, 3 et 7 à 9, comprenant en outre un cathéter (312, 611) dont une partie distale (324) comprend le premier élément allongé (326a, 612) et le deuxième élément allongé (326b, 622).

16. Ensemble de coupe (310) guidé par un fil selon la revendication 15, comprenant en outre un élément d'orientation (332, 333) prévu sur chacun des premier élément allongé (326a) et deuxième élément allongé (326b), afin de faciliter l'imagerie et/ou l'orientation rotative de l'ensemble de coupe (310) guidé par un fil pendant son utilisation.

17. Ensemble de coupe (10, 110) guidé par un fil selon la revendication 1, le premier élément allongé (12, 112) étant un cathéter, l'ensemble (22, 122) de coupe de tissu étant un élément coupant fixé en un lieu prédéterminé sur une périphérie d'une partie distale (24, 124) du cathéter, l'élément coupant étant configuré pour créer une incision linéaire dans une paroi d'une lumière corporelle, sensiblement parallèle à un axe longitudinale du cathéter, et le deuxième élément allongé (34, 134) étant une agrafe adjacente à l'élément coupant, permettant d'engager du tissu entre l'agrafe et une surface extérieure du cathéter, afin de maintenir l'élément coupant en contact avec le tissu.

18. Ensemble de coupe (10) guidé par un fil selon la revendication 17, comprenant en outre une gaine (40) servant à couvrir et à découvrir de manière choisie l'agrafe, la gaine (40) maintenant l'agrafe en contact intime avec la surface extérieure du cathéter quand la gaine (40) couvre l'agrafe flexible.

19. Ensemble de coupe (10, 110) guidé par un fil selon la revendication 17, dans lequel l'agrafe comprend une partie flexible servant à éloigner une extrémité libre (38, 138) de l'agrafe de la surface extérieure du premier élément allongé (12, 112) en la poussant, afin de faciliter l'engagement de tissu entre l'agrafe et la surface extérieure du premier élément allongé (12, 112).

20. Ensemble de coupe (10, 110) guidé par un fil selon la revendication 17, comprenant en outre un filament (140) relié avec possibilité de retrait à une extrémité libre (38, 138) de l'agrafe, le filament (140) s'étendant de façon proximale jusqu'à l'extrémité proximale du premier élément allongé (12, 112), moyennant quoi le filament (140) peut maintenir l'extrémité libre (38, 138) de l'agrafe en contact intime avec la surface extérieure du premier élément allongé (12, 112), jusqu'à ce que le filament (140) soit dégagé de l'extrémité proximale (14, 114) du premier élément allongé (12, 112).

21. Ensemble de coupe (10) guidé par un fil selon la revendication 17, dans lequel l'agrafe est au moins en partie rétractable dans le premier élément allongé (12).

22. Ensemble de coupe (10, 110) guidé par un fil selon la revendication 17, comprenant en outre un élément d'orientation (33, 133) sur l'agrafe, afin de suivre sa position par rapport à l'élément coupant.

23. Appareil (410) permettant de créer un canal entre des vaisseaux sanguins adjacents (82, 84), comprenant :
un cathéter (412) comprenant une première lumière (420) de guidage de fil et une deuxième lumière (421) de guidage de fil, qui s'étendent à partir d'une extrémité proximale ou d'une zone intermédiaire du cathéter (412) jusqu'à une partie distale (424) du cathéter (412),
la première lumière (420) de guidage de fil s'étendant axialement jusqu'à un premier orifice (420a) de sortie sur une extrémité distale (416) du cathéter (412),
la deuxième lumière (421) de guidage de fil comportant un deuxième orifice de sortie (421a) en un lieu proche de l'extrémité distale (416) du cathéter (412),
et un élément coupant (422) situé sur la partie distale (424) du cathéter (412), entre les premier et deuxième orifices de sortie (420a, 421a).
